# EUROPEAN PATENT APPLICATION

(11) **EP 2 851 425 A1**
(43) Date of publication of application: **25.03.2015**
(21) Application number: 13790416.5
(22) Date of filing: 13.05.2013
(51) Int. Cl.: C12N 15/09, C12Q 1/68

(54) **RNA DETECTION METHOD AND DETECTION KIT**

(30) Priority: 15.05.2012 JP 2012111644; 15.05.2012 JP 2012111645
(71) Applicant: Eisai R&D Management Co., Ltd., Tokyo 112-8088 (JP)
(72) Inventor: FUJIKAWA, Toshihiko, Inashiki-gun Ibaraki 300-1155 (JP); OSAWA, Masako, Inashiki-gun Ibaraki 300-1155 (JP)
(74) Representative: Grünecker, Kinkeldey, Stockmair & Schwanhäusser
(86) International application number: PCT/JP2013/063297
(87) International publication number: WO 2013/172305

(57) **Abstract**

Provided are a method and kit for detecting RNA, which allow RNA such as microRNA and messenger RNA to be detected with high sensitivity and simply, and signal amplification probes to be used in the method. Specifically, provided is a method of detecting target RNA, the method including: (A) a capturing step of capturing RNA by a capture substance for capturing the RNA; (B) a complex-for-detection forming step of subjecting the captured RNA and signal amplification probes capable of self-assembling to a reaction to form a complex for detection including the RNA, the capture substance, and a probe polymer formed from the signal amplification probes; and (C) a detecting step of detecting the probe polymer bound in the complex for detection to detect the RNA, one of the signal amplification probes including a poly(T) sequence.

SEQUENCE LISTING

<110>  Eisai R&D Management Co., Ltd.

<120>  Method for detecting RNA and detection kit

<130>  77981-P-PCT

<150>  JP 2012-111644
<151>  2012-05-15

<150>  JP 2012-111645
<151>  2012-05-15

<160>  25

<170>  PatentIn version 3.1

<210>  1
<211>  22
<212>  RNA
<213>  Artificial

<220>
<223>  Description of Artificial Sequence; Synthetic

<400>  1
aacccguaga uccgaacuug ug                                                22

<210>  2
<211>  22
<212>  RNA
<213>  Artificial

<220>
<223>  Description of Artificial Sequence; Synthetic

<400>  2
uuaaugcuaa ucgugauagg gg                                                22

<210>  3
<211>  22
<212>  RNA
<213>  Artificial

<220>
<223>  Description of Artificial Sequence; Synthetic

<400>  3
uagcagcaca ucaugguuua ca                                                22

<210>  4
<211>  22
<212>  RNA
<213>  Artificial

<220>
<223>  Description of Artificial Sequence; Synthetic

<400>  4

uagcagcacg uaaauauugg cg                                                22

<210>  5
<211>  22
<212>  RNA
<213>  Artificial

<220>
<223>  Description of Artificial Sequence; Synthetic

<400>  5
uagcuuauca gacugauguu ga                                                22

<210>  6
<211>  21
<212>  RNA
<213>  Artificial

<220>
<223>  Description of Artificial Sequence; Synthetic

<400>  6
aucacauugc cagggauuuc c                                                 21

<210>  7
<211>  22
<212>  DNA
<213>  Artificial

<220>
<223>  Description of Artificial Sequence; Synthetic

<400>  7
aactatgcaa cctactacct ct                                                22

<210>  8
<211>  22
<212>  DNA
<213>  Artificial

<220>
<223>  Description of Artificial Sequence; Synthetic

<400>  8
cacaagttcg gatctacggg tt                                                22

<210>  9
<211>  23
<212>  DNA
<213>  Artificial

<220>
<223>  Description of Artificial Sequence; Synthetic

<400>  9
tgatagccct gtacaatgct gct                                               23

<210>  10
<211>  22
<212>  DNA
<213>  Artificial

<220>
<223>  Description of Artificial Sequence; Synthetic

<400>  10
cccctatcac gattagcatt aa                                                22


<210>  11
<211>  22
<212>  DNA
<213>  Artificial

<220>
<223>  Description of Artificial Sequence; Synthetic

<400>  11
tgtaaaccat gatgtgctgc ta                                                22


<210>  12
<211>  22
<212>  DNA
<213>  Artificial

<220>
<223>  Description of Artificial Sequence; Synthetic

<400>  12
cgccaatatt tacgtgctgc ta                                                22


<210>  13
<211>  23
<212>  DNA
<213>  Artificial

<220>
<223>  Description of Artificial Sequence; Synthetic

<400>  13
actcaccgac agcgttgaat gtt                                               23


<210>  14
<211>  22
<212>  DNA
<213>  Artificial

<220>
<223>  Description of Artificial Sequence; Synthetic

<400>  14
actcaccgac aggttgaatg tt                                                22


<210>  15
<211>  22
<212>  DNA

<213>  Artificial

<220>
<223>  Description of Artificial Sequence; Synthetic

<400>  15
tcaacatcag tctgataagc ta                                              22


<210>  16
<211>  23
<212>  DNA
<213>  Artificial

<220>
<223>  Description of Artificial Sequence; Synthetic

<400>  16
gaaacccagc agacaatgta gct                                             23


<210>  17
<211>  21
<212>  DNA
<213>  Artificial

<220>
<223>  Description of Artificial Sequence; Synthetic

<400>  17
ggaaatccct ggcaatgtga t                                               21


<210>  18
<211>  22
<212>  DNA
<213>  Artificial

<220>
<223>  Description of Artificial Sequence; Synthetic

<400>  18
acaggccggg acaagtgcaa ta                                              22


<210>  19
<211>  46
<212>  DNA
<213>  Artificial

<220>
<223>  Description of Artificial Sequence; Synthetic

<400>  19
caacaatcag gacgataccg atgaagtttt tttttttttt tttttt                    46


<210>  20
<211>  46
<212>  DNA
<213>  Artificial

<220>

<223>  Description of Artificial Sequence; Synthetic

<400>  20
gtcctgattg ttgcttcatc ggtatcaaaa aaaaaaaaaa aaaaaa                46

<210>  21
<211>  61
<212>  DNA
<213>  Artificial

<220>
<223>  Description of Artificial Sequence; Synthetic

<400>  21
aaggtgtgca cttttattca actggtctca agtcagtgta caggtaagcc ctggctgcct      60

c                                                                     61

<210>  22
<211>  61
<212>  DNA
<213>  Artificial

<220>
<223>  Description of Artificial Sequence; Synthetic

<400>  22
ggttgagcac agggtacttt attgatggta catgacaagg tgcggctccc taggcccctc      60

c                                                                     61

<210>  23
<211>  61
<212>  DNA
<213>  Artificial

<220>
<223>  Description of Artificial Sequence; Synthetic

<400>  23
tggtggtgca ggaggcattg ctgatgatct tgaggctgtt gtcatacttc tcatggttca      60

c                                                                     61

<210>  24
<211>  60
<212>  DNA
<213>  Artificial

<220>
<223>  Description of Artificial Sequence; Synthetic

<400>  24
acccatgacg aacatggggg catcagcaga gggggcagag atgatgaccc ttttggctcc      60

<210>  25
<211>  60
<212>  DNA
<213>  Artificial

<220>
<223>  Description of Artificial Sequence; Synthetic

<400>  25
tgagtccttc cacgatacca aagttgtcat ggatgacctt ggccaggggt gctaagcagt      60

## Description

### Technical Field

The present invention relates to a method and kit for detecting RNA such as microRNA (miRNA) and messenger RNA (mRNA), and signal amplification probes to be used in the method, and more particularly, to a method and kit for detecting RNA, which allow RNA to be detected with high sensitivity and simply, and signal amplification probes.

### Background Art

MicroRNA is a kind of small RNA to be transcribed in various eukaryotes including humans. A primary transcript (pri-miRNA) transcribed from a non-gene region or intron portion on a genome is processed into precursor microRNA (pre-miRNA) having a hairpin structure and then into mature microRNA (mature-miRNA) having about 22 bases (Non Patent Document 1). It has been revealed that the microRNA functions as a regulatory factor in a process of translation from transcribed mRNA to a protein (Non Patent Document 2). At present, 800 or more kinds of microRNAs are expected to be present on a human genome (Non Patent Document 3), but many of the microRNAs have not been identified for a messenger (mRNA) subject to their regulatory functions.

However, previous research has revealed that many microRNAs regulate expression of genes involved in multicellular organism development, differentiation, cell growth, and the like (Non Patent Document 4). In addition, there are reports on microRNAs considered to function in canceration or cancer suppression mechanism of cells, and many of the microRNAs are expressed in increased or decreased amounts in cancer cells as compared to normal cells (Non Patent Document 5). Thus, it is considered that an investigation on an expression amount of microRNA enables profiling of canceration of a cell or tissue of interest to be performed in the future, and it is predicted that identification or quantitative determination of microRNA in a cell or a tissue serves as one of the important items for diagnosis of cancer in the future.

Heretofore, as methods of detecting microRNA, there have been reported a northern blotting method (Non Patent Document 6), a primer elongation method (Non Patent Documents 7 and 8), an invader method (Non Patent Document 9), a ribozyme signal amplification method (Non Patent Document 10), a mirMASA bead-based technology (Non Patent Document 11), bead-based flow cytometry (Non Patent Document 12), a real-time RT-PCR (Non Patent Documents 13 and 14), a microarray (Non Patent Document 15), and the like, and there has been attempted a labeling method involving using gold particles (Non Patent Document 16). In addition, a kit using an RNase protection assay is available as a reagent for research (Non Patent Document 17).

However, the above-mentioned methods have problems such as insufficient sensitivity (northern blotting method), necessity of amplifying precursor microRNA, which serves as a target, by a PCR (e.g., real-time RT-PCR), detection requiring much time and complicated operations (bead-based flow cytometry, invader method), and difficulty in simultaneously analyzing many kinds of target nucleic acids (primer elongation method, invader method).

Meanwhile, messenger RNA is a nucleic acid responsible for part of gene expression and is synthesized by a function of RNA polymerase using DNA as a template (transcription). After that, a protein of interest is synthesized by a ribosome using genetic information included in the messenger RNA (translation). The messenger RNA can be said to be a very important element for expression of a protein from DNA. Messenger RNA from eukaryotes has a CAP structure at the 5' end and poly(A) at the 3' end unlike other RNAs, i.e., transfer RNA (tRNA) and ribosomal RNA (rRNA). The structures also have a function of controlling decomposition of the messenger RNA itself.

Heretofore, in the field of diagnosis, a protein has been used as a biomarker mainly in a biochemical approach. However, it is difficult to prepare an antibody for detecting the protein in some cases, and the protein alone is not necessarily sufficient as the biomarker in terms of sensitivity and specificity. Thus, research has been made on whether or not expression of messenger RNA from which a protein originates can be used as a biomarker associated with a disease or the like. Studies on expression of several thousands of or several tens of thousands of messenger RNAs using a messenger array have revealed presence of an expression profile of messenger RNA specific for a certain disease. In addition, not only expression profiles obtained from many messenger RNAs but also single messenger RNA is known to serve as a biomarker. For example, as a myeloid leukemia-related diagnostic drug, messenger RNA of a certain gene is used as a biomarker, and is actually covered by insurance as an in vitro diagnostic in Japan. As can be seen from those facts, usefulness of messenger RNA as a next-generation biomarker serving as an alternative to a protein is considered to increase continuously in the future.

Heretofore, as methods of detecting messenger RNA, there have been reported methods involving using an RT-PCR method, a real-time PCR method including TaqMan, a messenger array, and a branched-DNA method.

However, the above-mentioned methods have problems such as necessity of a step of converting target messenger RNA from RNA to DNA by reverse transcription or necessity of amplifying the target messenger RNA by a PCR (e.g., real-time RT-PCR), detection requiring a time of 1 day or more and complicated operations (messenger array), and difficulty in simultaneously analyzing many kinds of target nucleic acids (real-time

PCR).

Meanwhile, in Patent Documents 1 and 2, there is a disclosure of a novel isothermal signal amplification method without using any enzyme. This method is a method involving subjecting a pair of oligonucleotide probes having base sequence regions complementary to each other to a self-assembly reaction to form an assembly of the probes (probe polymer), and is applied to detection of a target nucleic acid in a sample by quantitative determination of the polymer. This method is, for example, a method of effectively detecting a target nucleic acid, involving using a probe (assist probe) having both of a base sequence complementary to a target nucleic acid and a sequence complementary to a probe to be used in probe polymer formation, or using, as one site of a complementary base sequence region of a probe to be used, a base sequence complementary to a target nucleic acid, or allowing the probe and the target nucleic acid to directly or indirectly bind to each other to form a probe polymer, and is called a PALSAR method.

In addition, in Patent Document 3, there is a disclosure of a method of detecting a small nucleic acid including microRNA. However, this method has a problem in that its sensitivity is at a level of several hundred amol and hence limitations are imposed on sensitivity and a reaction temperature, although the method has an advantage such as no modification of microRNA.

Further, in Patent Document 4, although detection of messenger RNA is performed using a PALSAR method, it is essential to perform a reverse transcription reaction in this method.

### Prior Art Documents

### Patent Documents

Patent Document 1: JP 3267576 B
Patent Document 2: JP 3310662 B
Patent Document 3: WO 2010/087409 A1
Patent Document 4: WO 2004/072302 A1
Patent Document 5: WO 02/031192 A1
Patent Document 6: JP 2002-355081 A
Patent Document 7: WO 2009/54320 A1

### Non Patent Documents

Non Patent Document 1: Kim VN, Nam JW. Genomics of microRNA. Trends Genet. 2006 Mar; 22(3): 165-73.
Non Patent Document 2: Sontheimer, E., J. Assembly and function of RNA silencing complex. Nature Reviews Molecular Cell Biology. 2005 Feb; 6 127-138
Non Patent Document 3: Bentwich I, Avniel A, Karov Y, Aharonov R, Gilad S, Barad O, Barzilai A, Einat P, Einav U, Meiri E, Sharon E, Spector Y, Bentwich Z. Identification of hundreds of conserved and nonconserved human microRNAs. Nat Genet. 2005 Jul; 37(7): 766-70
Non Patent Document 4: Alvarez-Garcia I, Miska EA. MicroRNA functions in animal development and human disease. Development. 2005 Nov; 132(21): 4653-62.
Non Patent Document 5: Esquela-Kerscher A, Slack FJ. Oncomirs - microRNAs with a role in cancer. Nat Rev Cancer. 2006 Apr; 6(4): 259-269
Non Patent Document 6: Lagos-Quintana, M., Rauhut, R., Meyer, J., Borkhardt, A., and Tuschl, T. New microRNAs from mouse and human. RNA. 2003 Feb; 9(2): 175-179.
Non Patent Document 7: Zeng, Y. and Cullen, B.R. Sequence requirements for micro RNA processing and function in human cells. RNA. 2003 Jan; 9(1): 112-123.
Non Patent Document 8: Raymond CK, Roberts BS, Garrett-Engele P, Lim LP, Johnson JM. Simple, quantitative primer-extension PCR assay for direct monitoring of microRNAs and short-interfering RNAs. RNA. 2005 Nov; 11: 1737-1744
Non Patent Document 9: Allawi, H.T., Dahlberg, J.E., Olson, S., Lund, E., Olson, M., Ma, W.P., Takova, T., Neri, B.P., and Lyamichev, V.I. Quantitation of microRNAs using a modified Invader assay. RNA. 2004 Jul; 10(7): 1153-1161.
Non Patent Document 10: Hartig, J.S., Grune, I., Najafi-Shoushtari, S.H., and Famulok, M. Sequence-specific detection of MicroRNAs by signal-amplifying ribozymes. J. Am. Chem. Soc. 2004 Jan 28; 126(3): 722-723.
Non Patent Document 11: Barad O, Meiri E, Avniel A, Aharonov R, Barzilai A, Bentwich I, Einav U, Gilad S, Hurban P, Karov Y, Lobenhofer EK, Sharon E, Shiboleth YM, Shtutman M, Bentwich Z, Einat P. MicroRNA expression detected by oligonucleotide microarrays: system establishment and expression profiling in human tissues. Genome Res. 2004 Dec; 14(12): 2486-94.
Non Patent Document 12: Lu J, Getz G, Miska EA, Alvarez-Saavedra E, Lamb J, Peck D, Sweet-Cordero A, Ebert BL, Mak RH, Ferrando AA, Downing JR, Jacks T, Horvitz HR, Golub TR. MicroRNA expression profiles classify human cancers. Nature. 2005 Jun 9; 435(7043): 834-8
Non Patent Document 13: Chen C, Ridzon DA, Broomer AJ, Zhou Z, Lee DH, Nguyen JT, Barbisin M, Xu NL, Mahuvakar VR, Andersen MR, Lao KQ, Livak KJ, Guegler KJ. Real-time quantification of microRNAs by stem-loop RT-PCR. Nucleic Acids Res. 2005 Nov 27; 33(20): e179.
Non Patent Document 14: Tang F, Hajkova P, Barton SC, Lao K, Surani MA. MicroRNA expression profiling of single whole embryonic stem cells. Nucleic Acids Res. 2006 Jan 24; 34(2): e9.
Non Patent Document 15: Liu CG, Calin GA, Meloon B, Gamliel N, Sevignani C, Ferracin M, Dumitru CD, Shimizu M, Zupo S, Dono M, Alder H, Bullrich F, Negrini M, Croce CM. An oligonucleotide microchip for genome-wide microRNA profiling in human and mouse tissues. Proc Natl Acad Sci USA. 2004 Jan 29; 101(26): 9740-4.
Non Patent Document 16: Liang RQ, Li W, Li Y, Tan CY, Li JX, Jin YX, Ruan KC. An oligonucleotide microarray for microRNA expression analysis based on labeling RNA with quantum dot and nanogold probe. Nucleic Acids Res. 2005 Jan 31; 33(2): e17.
Non Patent Document 17: mirVana miRNA Detection kit (Ambion Inc.)

### Summary of the Invention

### Problem to be solved by the invention

An object of the present invention is to provide a method and kit for detecting RNA, which allow RNA such as microRNA and messenger RNA to be detected with high sensitivity and simply, and signal amplification probes to be used in the method.

### Means for solving problem

In order to achieve the above-mentioned object, the inventors of the present invention have made intensive studies. As a result, the inventors have surprisingly found that RNA can be detected with high sensitivity by: using in signal amplification probes for a hybridization reaction to be used in a PALSAR method, signal amplification probes in which one region of one of the signal amplification probes is a region including a poly(T) sequence, which is a nucleic acid sequence of consecutive thymines (T); adding poly(A), which is a nucleic acid sequence of consecutive adenines (A), to the 3' end of target RNA as necessary; then subjecting RNA captured by a capture probe and the signal amplification probes to a reaction to form a complex for detection including a probe polymer; and detecting the probe polymer.

That is, a method of detecting RNA of the present invention is a method of detecting target RNA, the method including: (A) a capturing step of capturing RNA capable of including a poly(A) sequence at the 3' end by a capture substance for capturing the RNA, i.e., a capturing step of capturing RNA by a capture substance for capturing the RNA; (B) a complex-for-detection forming step of subjecting the captured RNA and one kind or a plurality of kinds of signal amplification probes capable of self-assembling to a reaction to form a complex for detection including the RNA, the capture substance, and a probe polymer formed from the signal amplification probes; and (C) a detecting step of detecting the probe polymer bound in the complex for detection to detect the RNA, one of the signal amplification probes having a poly(T) sequence.

It is preferred that in the signal amplification probe, the poly(T) sequence have a length of from 11 to 26 bases.

It is suitable that the signal amplification probe having a poly(T) sequence have the poly(T) sequence at the 3' end.

It is preferred that at least one of the signal amplification probes be labeled with a labeling substance.

It is suitable that the capture substance include a base sequence complementary to the RNA, and be immobilized onto a support or have introduced therein a moiety capable of being immobilized onto the support.

In the present invention, the hybridization reaction is not particularly limited, but more suitably includes a self-assembly reaction in which nucleic acids self-assemble to form a double-stranded self-assembly through hybridization using a plurality of kinds of nucleic acid probes having complementary base sequence regions capable of hybridizing with each other.

That is, a first example of the method of detecting RNA of the present invention is a method of detecting target RNA, the method including: (A) a capturing step of capturing RNA by a capture substance for capturing the RNA; (B) a step of subjecting the captured RNA, a first signal amplification probe, and a second signal amplification probe to a reaction to form a complex for detection including the RNA, the capture substance, and a probe polymer formed from the first signal amplification probe and the second signal amplification probe; and (C) a step of detecting the probe polymer bound in the complex for detection to detect the RNA, the first signal amplification probe including a nucleic acid probe that includes at least a nucleic acid region X, a nucleic acid region Y, and a nucleic acid region Z including a poly(T) sequence in the stated order from the 5' end side, the second signal amplification probe including a nucleic acid probe that includes at least a nucleic acid region X' complementary to the nucleic acid region X, a nucleic acid region Y' complementary to the nucleic acid region Y, and a nucleic acid region Z' complementary to the nucleic acid region Z in the stated order from the 5' end side.

It is preferred that in the first signal amplification probe, the poly(T) sequence have a length of from 11 to 26 bases.

It is suitable that in the first signal amplification probe, the nucleic acid region X have a length of from 11 to 20 bases, the nucleic acid region Y have a length of from 11 to 20 bases, and the nucleic acid region Z have a length of from 11 to 26 bases.

It is preferred that the first signal amplification probe and/or the second signal amplification probe be labeled with a labeling substance.

In the present invention, any of RNA having a poly(A) sequence, which is a nucleic acid sequence of consecutive adenines (A), such as mRNA, and RNA having no poly(A) sequence may be detected as the target RNA. When the RNA having a poly(A) sequence is detected as the target RNA, the poly(A) sequence of the target RNA and the poly(T) sequence of the signal amplification probe hybridize with each other to form a complex for detection, and the complex for detection can be detected to detect the target RNA. In addition, when the RNA having no poly(A) sequence, for example, microRNA having no poly(A) sequence is detected as the target RNA, by performing (D) a poly(A) adding step of adding poly(A) to the 3' end of the microRNA before or after the step (A) (preferably before the step (A)), the poly(A) sequence of the poly(A)-added target RNA and the poly(T) sequence of the signal amplification probe hybridize with each other to form a complex for detection, and the complex for detection can be detected to detect the target RNA.

A kit for detecting RNA of the present invention is a kit for detecting RNA, the kit including: a capture substance for capturing target RNA; and one kind or a plurality of kinds of signal amplification probes, in which the plurality of kinds of signal amplification probes are a plurality of kinds of signal amplification probes that have complementary base sequence regions capable of hybridizing with each other and can form a probe polymer through a self-assembly reaction, one of the plurality of kinds of signal amplification probes having a poly(T) sequence, and in which the one kind of signal amplification probe is one kind of signal amplification probe having a poly(T) sequence and including three or more nucleic acid regions, each of the nucleic acid regions in the one kind of signal amplification probe including a first region and a second region complementary to the first region so that the regions are adjacent to each other.

It is suitable that the plurality of kinds of signal amplification probes include a first signal amplification probe and a second signal amplification probe, the first signal amplification probe be a nucleic acid probe that includes at least a nucleic acid region X, a nucleic acid region Y, and a nucleic acid region Z including a poly(T) sequence in the stated order from the 5' end side, and the second signal amplification probe be a nucleic acid probe that includes at least a nucleic acid region X' complementary to the nucleic acid region X, a nucleic acid region Y' complementary to the nucleic acid region Y, and a nucleic acid region Z' complementary to the nucleic acid region Z in the stated order from the 5' end side.

Signal amplification probes of the present invention are one kind or a plurality of kinds of signal amplification probes to be used in the method of detecting RNA of the present invention, in which the plurality of kinds of signal amplification probes are a plurality of kinds of signal amplification probes that have complementary base sequence regions capable of hybridizing with each other and can form a probe polymer through a self-assembly reaction, one of the plurality of kinds of signal amplification probes having a poly(T) sequence, and in which the one kind of signal amplification probe is one kind of signal amplification probe having a poly(T) sequence and including three or more nucleic acid regions, each of the nucleic acid regions in the one kind of signal amplification probe including a first region and a second region complementary to the first region so that the regions are adjacent to each other.

It is preferred that in the signal amplification probe, the poly(T) sequence have a length of from 11 to 26 bases.

It is suitable that the signal amplification probe having a poly(T) sequence have the poly(T) sequence at the 3' end.

It is preferred that at least one of the signal amplification probes be labeled with a labeling substance.

According to a first exemplary embodiment, the plurality of kinds of signal amplification probes are a pair of signal amplification probes, including a first signal amplification probe and a second signal amplification probe, in which the first signal amplification probe is a nucleic acid probe that includes at least a nucleic acid region X, a nucleic acid region Y, and a nucleic acid region Z including a poly(T) sequence in the stated order from the 5' end side, and in which the second signal amplification probe is a nucleic acid probe that includes at least a nucleic acid region X' complementary to the nucleic acid region X, a nucleic acid region Y' complementary to the nucleic acid region Y, and a nucleic acid region Z' complementary to the nucleic acid region Z in the stated order from the 5' end side.

According to a second exemplary embodiment, the plurality of kinds of signal amplification probes are a plurality of dimer probes or dimer formation probes for forming the dimer probes, in which each of the plurality of dimer probes is a dimer formed from two kinds of dimer formation probes, in which each of the dimer formation probes includes three regions, i.e., a 5' side region, a mid-region, and a 3' side region, in which in the two kinds of dimer formation probes, the mid-regions are complementary to each other and the 3' side regions and the 5' side regions are non-complementary to each other, and in which each of the 5' side regions of each of the plurality of dimer probes is complementary to any one of the 5' side regions of the other dimer probes and each of the 3' side regions of each of the dimer probes is complementary to any one of the 3' side regions of the other dimer probes.

It is suitable that in the signal amplification probes, the 5' side region have a length of from 11 to 20 bases, the mid-region have a length of from 11 to 20 bases, and the 3' side region have a length of from 11 to 26 bases.

According to a third exemplary embodiment, the plurality of kinds of signal amplification probes include one or more pairs of dimer formation probes or one or more dimer probes to be formed from the pairs of dimer formation probes, and one or more cross-linking probes, in which each of the dimer formation probes includes three regions, i.e., a 5' side region, a mid-region, and a 3' side region, the mid-regions of each of the pairs of dimer formation probes are complementary to each other, and all of the 3' side regions and 5' side regions of each of the pairs of dimer formation probes are non-complementary to each other, and in which each of the cross-linking probes includes two regions, i.e., a 5' side region and a 3' side region, the 3' side region of each of the cross-linking probes is complementary to any one of the 3' side regions of the dimer formation probes, and the 5' side region of each of the cross-linking probes is complementary to any one of the 5' side regions of the dimer formation probes.

It is suitable that in the signal amplification probes, the 5' side region have a length of from 11 to 20 bases, the mid-region have a length of from 11 to 20 bases, and the 3' side region have a length of from 11 to 26 bases.

### Effect of the Invention

According to one embodiment of the present invention, the method and kit for detecting RNA, which allow target RNA to be detected with high sensitivity and simply, and the signal amplification probes for a hybridization reaction to be used in the method can be provided. The present invention does not require a reverse transcription reaction and does not require an assist probe for linking a probe polymer and target RNA, and thus allows target RNA to be detected simply. According to another embodiment of the present invention, a plurality of kinds of target RNAs such as microRNA and mRNA can be simultaneously detected.

### Brief Description of Drawings

FIGS. 1 are schematic explanatory diagrams illustrating an example of a method of detecting RNA according to a first aspect of the present invention, FIG. 1(a) illustrates messenger RNA captured by a capture substance after a capturing step, and FIG. 1(b) illustrates a complex for detection after a complex-for-detection forming step.
FIG. 2 is a schematic explanatory diagram illustrating an example of signal amplification probes according to a first exemplary embodiment to be used in probe polymer formation.
FIG. 3 is a schematic explanatory diagram illustrating a probe polymer to be formed using the signal amplification probes illustrated in FIG. 2.
FIGS. 4 are schematic explanatory diagrams illustrating a first example of signal amplification probes according to a second exemplary embodiment to be used in probe polymer formation.
FIG. 5 is a schematic explanatory diagram illustrating a probe polymer to be formed using the signal amplification probes illustrated in FIGS. 4.
FIG. 6 is a schematic explanatory diagram illustrating a second example of the signal amplification probes according to the second exemplary embodiment to be used in probe polymer formation.
FIGS. 7 are schematic explanatory diagrams illustrating a third example of the signal amplification probes according to the second exemplary embodiment to be used in probe polymer formation.
FIGS. 8 are schematic explanatory diagrams illustrating a first example of signal amplification probes according to a third exemplary embodiment to be used in probe polymer formation.
FIG. 9 is a schematic explanatory diagram illustrating a probe polymer to be formed using the signal amplification probes illustrated in FIGS. 8.
FIG. 10 is a schematic explanatory diagram illustrating a second example of the signal amplification probes according to the third exemplary embodiment to be used in probe polymer formation.
FIG. 11 is a schematic explanatory diagram illustrating a probe polymer to be formed using the signal amplification probes illustrated in FIG. 10.
FIGS. 12 are schematic explanatory diagrams illustrating a third example of the signal amplification probes according to the third exemplary embodiment to be used in probe polymer formation.
FIG. 13 is a schematic explanatory diagram illustrating a probe polymer to be formed using the signal amplification probes illustrated in FIGS. 12.
FIGS. 14 are schematic explanatory diagrams illustrating a fourth example of the signal amplification probes according to the third exemplary embodiment to be used in probe polymer formation.
FIG. 15 is a schematic explanatory diagram illustrating an example of a signal amplification probe according to a fourth exemplary embodiment to be used in probe polymer formation.
FIGS. 16 are schematic explanatory diagrams illustrating the binding manner of the signal amplification probes illustrated in FIG. 15.
FIG. 17 is a schematic explanatory diagram illustrating a probe polymer to be formed using the signal amplification probes illustrated in FIG. 15.
FIGS. 18 are schematic explanatory diagrams illustrating an example of a method of detecting RNA according to a second aspect of the present invention, FIG. 18(a) illustrates microRNA, FIG. 18(b) illustrates poly(A)-added microRNA after a poly(A) adding step, FIG. 18(c) illustrates microRNA captured by a capture substance after a capturing step, and FIG. 18(d) illustrates a complex for detection after a complex-for-detection forming step.
FIGS. 19 are graphs showing the results of Example 1.
FIGS. 20 are graphs showing the results of Comparative Example 1.
FIG. 21 is a graph showing the results of Example 2.
FIG. 22 is a graph showing the results of Comparative Example 2.
FIG. 23 is a graph showing a correlation between the results of Example 2 and Comparative Example 2.
FIG. 24 is a graph showing the results of Example 3.
FIG. 25 is a graph showing the results of Example 4.
FIG. 26 is a graph showing the results of Example 5.

### Best modes for carrying out the invention

Embodiments of the present invention are described below with reference to the accompanying drawings. It should be appreciated that the illustrated examples are given for illustrative purposes only and various modifications may be made without departing from the technical concept of the present invention.

A method of detecting RNA of the present invention is a method of detecting target RNA, the method including: (A) a capturing step of capturing RNA by a capture substance for capturing the RNA; (B) a complex-for-detection forming step of subjecting the captured RNA and one kind or a plurality of kinds of signal amplification probes capable of self-assembling to a reaction to form a complex for detection including the RNA, the capture substance, and a probe polymer formed from the signal amplification probes; and (C) a detecting step of detecting the probe polymer bound in the complex for detection to detect the RNA, one of the signal amplification probes having a poly(T) sequence.

In the present invention, any of RNA having a poly(A) sequence, which is a nucleic acid sequence of consecutive adenines (A), such as mRNA, and RNA having no poly(A) sequence may be detected as the target RNA. When the RNA having a poly(A) sequence is detected as the target RNA, the poly(A) sequence of the target RNA and the poly(T) sequence of the signal amplification probe hybridize with each other to form a complex for detection, and the complex for detection can be detected to detect the target RNA. In addition, when the RNA having no poly(A) sequence, for example, microRNA having no poly(A) sequence is detected as the target RNA, by performing (D) a poly(A) adding step of adding poly(A) to the 3' end of the microRNA before or after the step (A) (preferably before the step (A)), the poly(A) sequence of the poly(A)-added target RNA and the poly(T) sequence of the signal amplification probe hybridize with each other to form a complex for detection, and the complex for detection can be detected to detect the target RNA.

As a method of detecting RNA according to a first aspect of the present invention, a case where the target RNA is messenger RNA having a poly(A) tail is described below.

The method of detecting RNA according to the first aspect of the present invention, in which the target RNA is messenger RNA having a poly(A) tail, is a method of detecting messenger RNA, the method including: (A) a capturing step of subjecting messenger RNA having a poly(A) tail and a capture substance for capturing the messenger RNA to a reaction to capture the messenger RNA; (B) a complex-for-detection forming step of subjecting the captured messenger RNA and one kind or a plurality of kinds of signal amplification probes to a reaction to form a complex for detection including the messenger RNA, the capture substance, and a probe polymer formed from the signal amplification probes; and (C) a detecting step of detecting the probe polymer bound in the complex for detection to detect the messenger RNA, the plurality of kinds of signal amplification probes being a plurality of kinds of signal amplification probes that have complementary base sequence regions capable of hybridizing with each other and can form a probe polymer through a self-assembly reaction, one of the plurality of kinds of signal amplification probes having a poly(T) sequence, the one kind of signal amplification probe being one kind of signal amplification probe having a poly(T) sequence and including three or more nucleic acid regions, each of the nucleic acid regions in the one kind of signal amplification probe including a first region and a second region complementary to the first region so that the regions are adjacent each other.

According to a first exemplary embodiment of the present invention, RNA is detected using a pair of signal amplification probes including a first signal amplification probe and a second signal amplification probe.

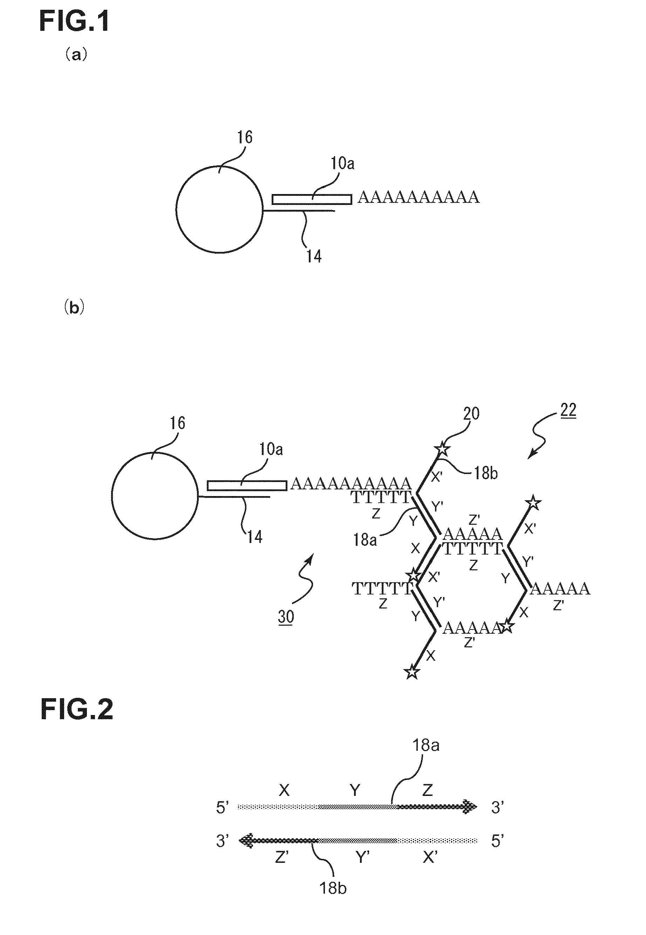

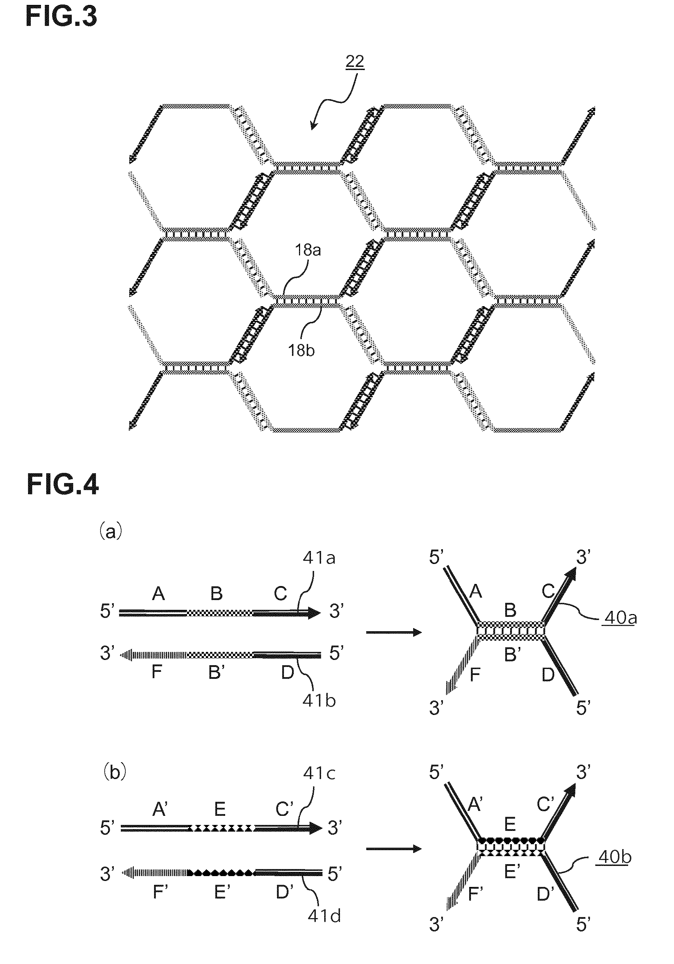

FIGS. 1 are schematic explanatory diagrams illustrating an example of the method of detecting RNA according to the first aspect of the present invention, FIG. 1(a) illustrates messenger RNA captured by a capture substance after a capturing step, and FIG. 1(b) illustrates a complex for detection after a complex-for-detection forming step. FIG. 2 is a schematic explanatory diagram illustrating an example of a pair of signal amplification probes to be used in probe polymer formation. FIG. 3 is a schematic explanatory diagram illustrating a probe polymer to be formed using the pair of signal amplification probes illustrated in FIG. 2.

In FIGS. 1, reference symbol 10a denotes messenger RNA. The present invention is suitably used for the detection of messenger RNA having poly(A) at the 3' end, is suitably used for the detection of messenger RNA having from 300 to 7,000 bases, and is particularly suitably used for the detection of messenger RNA having from about 300 to 3,000 bases. In addition, many kinds of nucleic acid molecules can be simultaneously detected.

As illustrated in FIG. 1(a), a capturing step of subjecting messenger RNA 10a having poly(A) and a capture substance 14 for capturing the target messenger RNA 10a to a reaction to capture the messenger RNA 10a is performed [step (A)].

The capture substance 14 may be any substance capable of specifically binding to the target messenger RNA without any particular limitation. However, it is suitable that the capture substance 14 include a base sequence complementary to the target messenger RNA 10a, and be immobilized onto a support 16 or have introduced therein a moiety capable of being immobilized onto the support 16.

It is preferred to use, as the support 16, for example, a fluorescent microparticle, a magnetic particle, a microplate, a microarray, a slide glass, or a substrate such as an electrically conductive substrate. It should be noted that in FIGS. 1, there is illustrated an example in the case of using a microparticle, but in the present invention, the support 16 is not particularly limited. It should be noted that in FIGS. 1, there is illustrated an example in which the support 16 is directly bound to a nucleic acid probe for capture including a base sequence complementary to the target messenger RNA 10a, but the support 16 may be bound to the nucleic acid probe for capture via any other nucleic acid probe, organic matter, or the like. For example, it may be possible to configure the nucleic acid probe for capture so as to further have a capture region K, and use the support 16 having immobilized thereonto a second capture probe having a region K' complementary to the capture region K.

Next, as illustrated in FIG. 1(b), a complex-for-detection forming step of subjecting the captured messenger RNA 10a, a first signal amplification probe 18a, and a second signal amplification probe 18b to a reaction to form a complex for detection 30 including the messenger RNA 10a, the capture substance 14, and a probe polymer 22 formed from the first signal amplification probe 18a and the second signal amplification probe 18b is performed [step (B)], and then a detecting step of detecting the probe polymer 22 bound in the complex for detection 30 to detect the messenger RNA is performed [step (C)] to detect the messenger RNA.

As illustrated in FIG. 2 and FIG. 1(b), the first signal amplification probe 18a is a nucleic acid probe that includes at least a nucleic acid region X, a nucleic acid region Y, and a nucleic acid region Z including a poly(T) sequence in the stated order from the 5' end side, and the second signal amplification probe 18b is a nucleic acid probe that includes at least a nucleic acid region X' complementary to the nucleic acid region X, a nucleic acid region Y' complementary to the nucleic acid region Y, and a nucleic acid region Z' complementary to the nucleic acid region Z in the stated order from the 5' end side. As illustrated in FIG. 3, when the first signal amplification probe 18a and the second signal amplification probe 18b are subjected to a reaction, the first signal amplification probe 18a and the second signal amplification probe 18b self-assemble to form an assembly (probe polymer 22).

The first signal amplification probe 18a to be used in the present invention has the nucleic acid region Z including a poly(T) sequence, and hence can bind to the messenger RNA 10a having a poly(A) tail. Thus, as illustrated in FIG. 1(b), the captured messenger RNA 10a having a poly(A) tail, the first signal amplification probe 18a, and the second signal amplification probe 18b are subjected to a reaction to form the complex for detection 30 including the messenger RNA 10a, the capture substance 14, and the probe polymer 22 formed from the first signal amplification probe 18a and the second signal amplification probe 18b, and the probe polymer 22 can be detected to detect the messenger RNA with high sensitivity and simply. A method of detecting the probe polymer is not particularly limited, and the probe polymer is suitably detected by means of, for example, fluorescence, luminescence, color development, or the like.

It should be noted that in FIGS. 1, there is illustrated an example using the capture substance 14 immobilized onto the support 16 in advance, but the timing of immobilizing the capture substance 14 onto the support 16 is not particularly limited, and the capture substance 14 and the support 16 may be allowed to directly or indirectly bind to each other during or after complex formation.

The length of each nucleic acid region in the first signal amplification probe 18a and the second signal amplification probe 18b is not particularly limited, but is preferably 5 bases or more, more preferably 8 bases or more, and it is particularly preferred that the nucleic acid regions X and X' each have a length of from 11 to 20 bases, the nucleic acid regions Y and Y' each have a length of from 11 to 20 bases, and the nucleic acid regions Z and Z' each have a length of from 11 to 26 bases. The lengths of the nucleic acid regions in the respective probes may be the same as or different from each other, but are desirably the same. In addition, in FIGS. 1 to FIG. 3, there is illustrated an example using a pair of signal amplification probes in which the number of complementary nucleic acid regions is 3, but a pair of signal amplification probes in which the number of complementary nucleic acid regions is 4 or more may be used.

As signal amplification probes according to a second exemplary embodiment to be used in the present invention, there are given a plurality of dimer probes capable of self-assembling or dimer formation probes for forming the dimer probes. The plurality of dimer probes are configured so that: each of the 5' side regions of one of the plurality of dimer probes is complementary to any one of the 5' side regions of the other dimer probes; and each of the 3' side regions of one of the plurality of dimer probes is complementary to any one of the 3' side regions of the other dimer probes. Thus, the plurality of dimer probes can self-assemble in themselves to form an assembly (probe polymer). For example, a nucleic acid probe disclosed in Patent Document 5 is used.

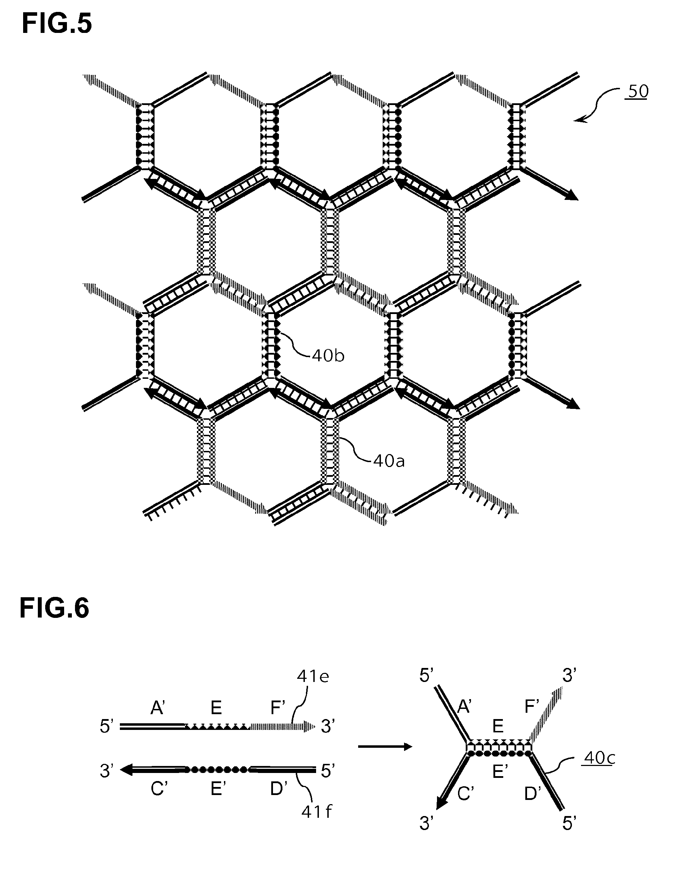

In FIGS. 4 and FIG. 5, there is illustrated an example of a case of using two dimer probes (a first dimer probe 40a and a second dimer probe 40b).

As illustrated in FIG. 4(a), the first dimer probe 40a is formed by allowing two kinds of single-stranded nucleic acid probes (a first dimer formation probe 41a and a second dimer formation probe 41b) to hybridize with each other. The first dimer formation probe 41a includes three regions, i.e., a 5' side region (region A), a mid-region (region B), and a 3' side region (region C), and the second dimer formation probe 41b includes three regions, i.e., a 5' side region (region D), a mid-region (region B'), and a 3' side region (region F). In the first dimer formation probe 41a and the second dimer formation probe 41b, the mid-regions (regions B and B') are complementary to each other, and the 3' side regions (regions C and F) and the 5' side regions (regions A and D) are non-complementary to each other.

As illustrated in FIG. 4(b), the second dimer probe 40b is formed by allowing two kinds of single-stranded nucleic acid probes (a third dimer formation probe 41c and a fourth dimer formation probe 41d) to hybridize with each other. The third dimer formation probe 41c includes three regions, i.e., a 5' side region (region A'), a mid-region (region E), and a 3' side region (region C'), and the fourth dimer formation probe 41d includes three regions, i.e., a 5' side region (region D'), a mid-region (region E'), and a 3' side region (region F'). In the third dimer formation probe 41c and the fourth dimer formation probe 41d, the mid-regions (regions E and E') are complementary to each other, and the 3' side regions (regions C' and F') and the 5' side regions (regions A' and D') are non-complementary to each other.

It should be noted that in the present invention, the region A' means a region having a base sequence complementary to the region A, the region C' means a region having a base sequence complementary to the region C, the region D' means a region having a base sequence complementary to the region D, and the region F' means a region having a base sequence complementary to the region F.

The 5' side regions (regions A and D) of the first dimer probe 40a are complementary to the 5' side regions (regions A' and D') of the second dimer probe 40b, and the 3' side regions (regions C and F) of the first dimer probe 40a are complementary to the 3' side regions (regions C' and F') of the second dimer probe 40b. Thus, the first and second dimer probes 40a and 40b can be allowed to hybridize with each other to form a signal probe polymer 50 (FIG. 5).

In FIGS. 4, there is illustrated an example in which: the 5' side region and 3' side region of the first dimer formation probe 41a are complementary to the 5' side region and 3' side region of the third dimer formation probe 41c, respectively; and the 5' side region and 3' side region of the second dimer formation probe 41b are complementary to the 5' side region and 3' side region of the fourth dimer formation probe 41d, respectively. In the present invention, however, the dimer probes only need to be configured so that: the 5' side region of one dimer probe is complementary to the 5' side region of another dimer probe; and the 3' side region of one dimer probe is complementary to the 3' side region of another dimer probe.

FIG. 6 is a schematic explanatory diagram illustrating another example (second example) of the second dimer probe to be used together with the first dimer probe 40a illustrated in FIGS. 4.

As illustrated in FIG. 6, a dimer probe 40c formed by allowing a dimer formation probe 41e, whose 5' side region is a region (region A') complementary to the 5' side region of the first dimer formation probe 41a illustrated in FIGS. 4 and whose 3' side region is a region (region F') complementary to the 3' side region of the second dimer formation probe 41b illustrated in FIGS. 4, and a dimer formation probe 41f, whose 5' side region is a region (region D') complementary to the 5' side region of the second dimer formation probe 41b illustrated in FIGS. 4 and whose 3' side region is a region (region C') complementary to the 3' side region of the first dimer formation probe 41a illustrated in FIGS. 4, to hybridize with each other may also be used as the second dimer probe.

In addition, in FIGS. 4, there is illustrated an example of using two kinds of dimer probes, but more kinds of dimer probes may also be used by devising a positional relationship between complementary regions (Patent Document 5).

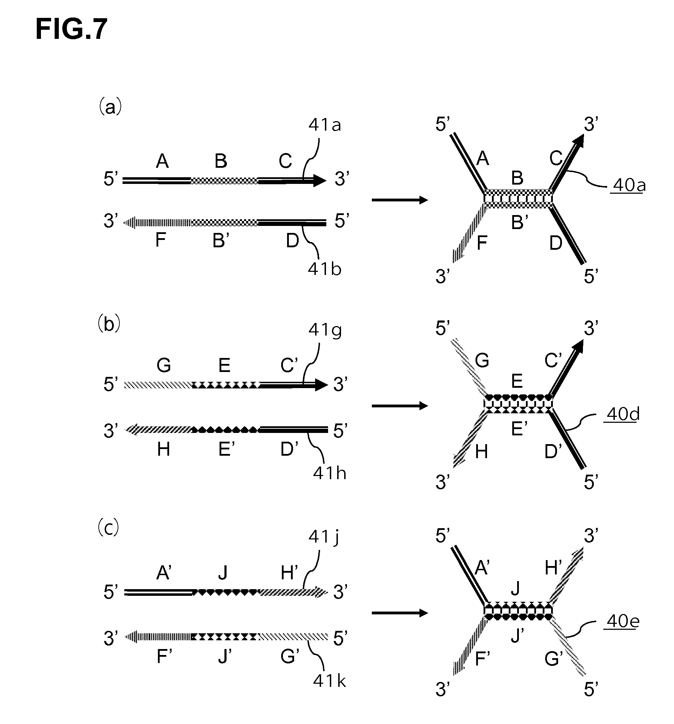

FIGS. 7 are schematic explanatory diagrams illustrating an example (third example) of a case of using three dimer probes (the first dimer probe 40a, a second dimer probe 40d, a third dimer probe 40e).

In FIG. 7(a), the first dimer probe 40a is configured in the same manner as in FIG. 4(a).

In FIG. 7(b), the second dimer probe 40d is formed by allowing two kinds of single-stranded nucleic acid probes (dimer formation probes 41g, 41h) to hybridize with each other. The dimer formation probe 41g includes three regions, i.e., a 5' side region (region G), a mid-region (region E), and a 3' side region (region C'), in which the 3' side region (region C') is complementary to the 3' side region (region C) of the first dimer probe 40a. The dimer formation probe 41h includes three regions, i.e., a 5' side region (region D'), a mid-region (region E'), and a 3' side region (region H), in which the 5' side region (region D') is complementary to the 5' side region (region D) of the first dimer probe 40a. The mid-region E of the dimer formation probe 41g is complementary to the mid-region E' of the dimer formation probe 41h.

In FIG. 7(c), the third dimer probe 40e is formed by allowing two kinds of single-stranded nucleic acid probes (dimer formation probes 41j, 41k) to hybridize with each other. The dimer formation probe 41j includes three regions, i.e., a 5' side region (region A'), a mid-region (region J), and a 3' side region (region H'), in which: the 5' side region (region A') is complementary to the 5' side region (region A) of the first dimer probe 40a; and the 3' side region (region H') is complementary to the 3' side region (region H) of the second dimer probe 40d. The dimer formation probe 41k includes three regions, i.e., a 5' side region (region G'), a mid-region (region J'), and a 3' side region (region F'), in which: the 5' side region (region G') is complementary to the 5' side region (region G) of the second dimer probe 40d; and the 3' side region (region F') is complementary to the 3' side region (region F) of the first dimer probe 40a. It should be noted that in FIG. 7(c), the region J' is a region complementary to the region J.

That is, in FIGS. 7, there is adopted such a configuration that: one 5' side region and one 3' side region of the first dimer probe are complementary to one 5' side region and one 3' side region of the second dimer probe, respectively; the other 5' side region and the other 3' side region of the first dimer probe are complementary to one 5' side region and one 3' side region of the third dimer probe, respectively; and the other 5' side region and the other 3' side region of the second dimer probe are complementary to the other 5' side region and the other 3' side region of the third dimer probe, respectively.

As illustrated in FIGS. 7, a signal probe polymer as an assembly of dimer probes is formed by: using a plurality of kinds of dimer probes configured so that each of the 3' side regions of each of the dimer probes is complementary to any one of the 3' side regions of the other dimer probes, and each of the 5' side regions of each of the dimer probes is complementary to any one of the 5' side regions of the other dimer probes; and allowing these plurality of kinds of dimer probes to hybridize with each other.

It should be noted that in the present invention, a combination of dimer probes having a complementary relationship is not particularly limited, but as illustrated in FIGS. 7, it is preferred to adopt such a configuration that one 3' side region and one 5' side region in each of the dimer probes are complementary to one 3' side region and one 5' side region in any one of the other dimer probes, respectively.

The length of each complementary region of the dimer formation probes is at least 5 bases, preferably at least 8 bases, more preferably from 10 bases to 100 bases, still more preferably from 12 to 30 bases in terms of the number of bases. In addition, the lengths of the complementary regions in the respective probes are desirably the same.

In the present invention, the dimer formation probes before dimer probe formation may be used in place of the dimer probes, but the dimer probes are preferably used. It should be noted that any one of the 3' regions (e.g., regions C, F, H) of the signal amplification probes according to the second exemplary embodiment includes a poly(T) sequence, and a region complementary thereto (e.g., regions C', F', H') includes a poly(A) sequence.

As signal amplification probes according to a third exemplary embodiment to be used in the present invention, there are given a plurality of kinds of signal amplification probes including one or more pairs of dimer formation probes, or one or more dimer probes to be formed from the pairs of dimer formation probes, and one or more kinds of cross-linking probes. The signal amplification probes according to a third exemplary embodiment are configured so that: each of the dimer formation probes includes three regions, i.e., a 5' side region, a mid-region, and a 3' side region, the mid-regions of each of the pairs of dimer formation probes are complementary to each other, and all of the 3' side regions and 5' side regions of each of the pairs of dimer formation probes are non-complementary to each other; each of the cross-linking probes includes two regions, i.e., a 5' side region and a 3' side region, each of the 5' side regions of the dimer formation probes is complementary to any one of the 5' side regions of the cross-linking probes, and each of the 3' side regions of the dimer formation probes is complementary to any one of the 3' side regions of the cross-linking probes; and the cross-linking probes can cross-link dimers to be formed from the dimer formation probes. Thus, the signal amplification probes can self-assemble in themselves to form an assembly (probe polymer). For example, a nucleic acid probe disclosed in Patent Document 6 is used.

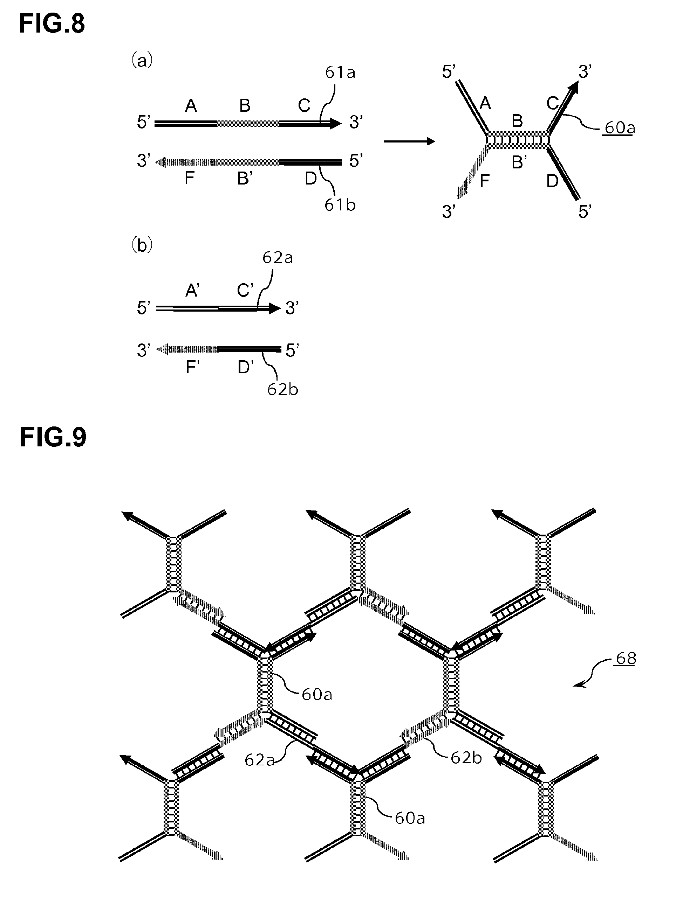

In FIGS. 8, there is illustrated a first example of a case of using a pair of dimer formation probes and a pair of cross-linking probes.

In FIG. 8(a), there are illustrated a pair of dimer formation probes (a first dimer formation probe 61a and a second dimer formation probe 61b), and a dimer probe 60a formed from the pair of dimer formation probes 61a, 61b.

As illustrated in FIG. 8(a), the pair of dimer formation probes is formed of two kinds of single-stranded nucleic acid probes (the first dimer formation probe 61a and the second dimer formation probe 61b), and each of the dimer formation probes 61a, 61b includes at least three regions, i.e., a mid-region, a 5' side region located closer to the 5' side with respect to the mid-region, and a 3' side region located closer to the 3' side with respect to the mid-region. In FIGS. 8, the 5' side region, mid-region, and 3' side region of the first dimer formation probe 61a are represented by a region A, a region B, and a region C, respectively, and the 5' side region, mid-region, and 3' side region of the second dimer formation probe 61b are represented by a region D, a region B', and a region F, respectively. The mid-regions (regions B and B') of the first dimer formation probe 61a and the second dimer formation probe 61b are complementary to each other, and all of the four regions, i.e., the 5' side regions (regions A and D) and 3' side regions (regions C and F) of the first dimer formation probe 61a and the second dimer formation probe 61b are non-complementary to each other. Both the probes are allowed to hybridize with each other to form the dimer probe 60a.

In the present invention, a dimer probe formed by allowing a pair of dimer formation probes to hybridize with each other in advance may be used, or the dimer formation probes may be used as they are, but the dimer probe is preferably used.

In the case of using a plurality of pairs of dimer formation probes, each of the pairs of dimer formation probes is configured in the same manner as in the case of using one pair of dimer formation probes described above. The plurality of pairs of dimer formation probes are used to form the same number of dimer probes.

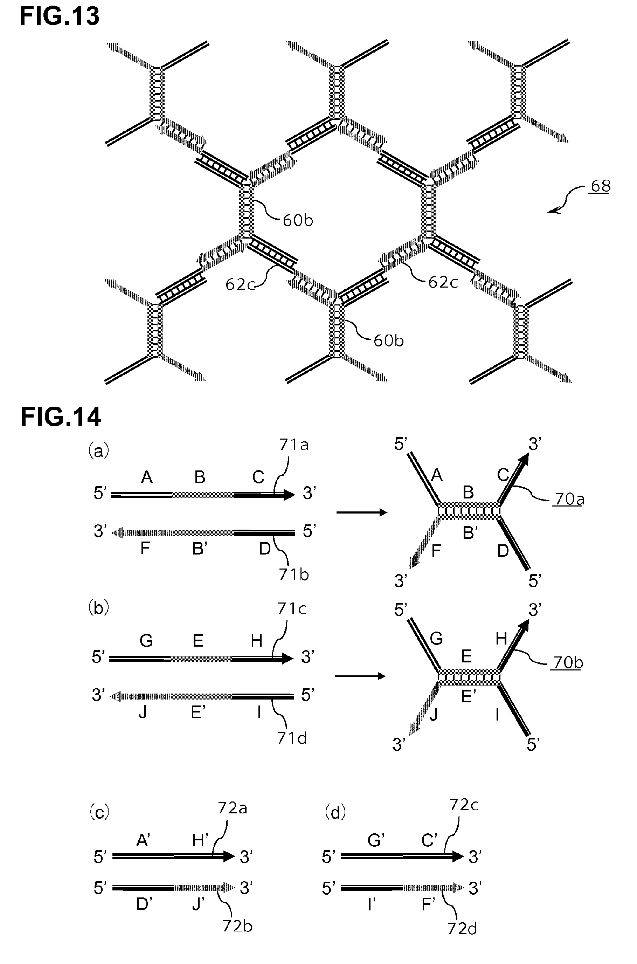

FIGS. 14(a) and 14(b) are schematic explanatory diagrams illustrating an example of two pairs of dimer formation probes, and two dimer probes to be formed from the pairs of dimer formation probes. FIG. 14(a) illustrates a first pair of dimer formation probes (a first dimer formation probe 71a and a second dimer formation probe 71b), and a dimer probe 70a to be formed from the pair of dimer formation probes 71a, 71b, and FIG. 14(b) illustrates a second pair of dimer formation probes (a first dimer formation probe 71c and a second dimer formation probe 71d), and a dimer probe 70b to be formed from the pair of dimer formation probes 71c, 71d.

In FIGS. 14, the 5' side region, mid-region, and 3' side region of the first dimer formation probe 71a in the first pair are represented by a region A, a region B, and a region C, respectively, the 5' side region, mid-region, and 3' side region of the second dimer formation probe 71b in the first pair are represented by a region D, a region B', and a region F, respectively, the 5' side region, mid-region, and 3' side region of the first dimer formation probe 71c in the second pair are represented by a region G, a region E, and a region H, respectively, and the 5' side region, mid-region, and 3' side region of the second dimer formation probe 71d in the second pair are represented by a region I, a region E', and a region J, respectively. The mid-regions (regions B and B', or regions E and E') of the first and second dimer formation probes in each of the pairs are complementary to each other, all of the four regions (regions A, C, D, and F, and regions G, H, I, and J), i.e., the 5' side regions and 3' side regions of the first and second dimer formation probes in the respective pairs are non-complementary to each other, and the dimer formation probes 71a, 71b, 71c, 71d in each of the pairs are allowed to hybridize with each other to form the two dimer probes 70a, 70b. In the present invention, it is preferred that all of the 3' side regions and 5' side regions of the dimer formation probes to be used be non-complementary to each other.

In the present invention, the cross-linking probes are one or more kinds of single-stranded nucleic acid probes that can cross-link the dimer probes to be formed from the dimer formation probes, and include at least two regions. Of the two regions, the region located on the 5' side is referred to as 5' side region, and the region located on the 3' side is referred to as 3' side region. In the present invention, each of the 5' side regions of the dimer formation probes is complementary to any one of the 5' side regions of the cross-linking probes, and each of the 3' side regions of the dimer formation probes is complementary to any one of the 3' side regions of the cross-linking probes. With such configuration, the cross-linking probes can bind to the dimer formation probes so as to cross-link one or more kinds of plurality of dimer probes to be formed from the dimer formation probes, to thereby form an assembly of probes (probe polymer).

In the case of using a pair of dimer formation probes, it is preferred to use a pair of cross-linking probes. The pair of dimer formation probes (a first dimer formation probe and a second dimer formation probe) and the pair of cross-linking probes (a first cross-linking probe and a second cross-linking probe) are configured so that: each of the 5' side regions of the pair of dimer formation probes is complementary to any one of the 5' side regions of the pair of cross-linking probes; each of the 5' side regions of the pair of cross-linking probes is complementary to any one of the 5' side regions of the pair of dimer formation probes; each of the 3' side regions of the pair of dimer formation probes is complementary to any one of the 3' side regions of the pair of cross-linking probes; and each of the 3' side regions of the pair of cross-linking probes is complementary to any one of the 3' side regions of the pair of dimer formation probes.

FIG. 8(b) is a schematic explanatory diagram illustrating an example of a pair of cross-linking probes (a first cross-linking probe 62a and a second cross-linking probe 62b) to be used together with the pair of dimer formation probes 61a, 61b illustrated in FIG. 8(a).

Suitable examples of the cross-linking probes to be used together with the pair of dimer formation probes 61a, 61b illustrated in FIG. 8(a) include, as illustrated in FIG. 8(b), such a pair of cross-linking probes that: the 5' side region of the first cross-linking probe 62a is complementary to the 5' side region (region A) of the first dimer formation probe 61a; the 3' side region of the first cross-linking probe 62a is complementary to the 3' side region (region C) of the first dimer formation probe 61a; the 5' side region of the second cross-linking probe 62b is complementary to the 5' side region (region D) of the second dimer formation probe 61b; and the 3' side region of the second cross-linking probe 62b is complementary to the 3' side region (region F) of the second dimer formation probe 61b. The dimer formation probes 61a, 61b illustrated in FIG. 8(a) and the cross-linking probes 62a, 62b illustrated in FIG. 8(b) can be allowed to hybridize with each other to form a signal probe polymer 68 (FIG. 9).

In FIGS. 8, there is illustrated an example in which: the 5' side region and 3' side region of the first dimer formation probe 61a are complementary to the 5' side region and 3' side region of the first cross-linking probe 62a, respectively; and the 5' side region and 3' side region of the second dimer formation probe 61b are complementary to the 5' side region and 3' side region of the second cross-linking probe 62b, respectively. In the present invention, such a configuration that the 5' side region of one dimer formation probe is complementary to the 5' side region of one cross-linking probe and the 3' side region of one dimer formation probe is complementary to the 3' side region of one cross-linking probe only needs to be adopted.

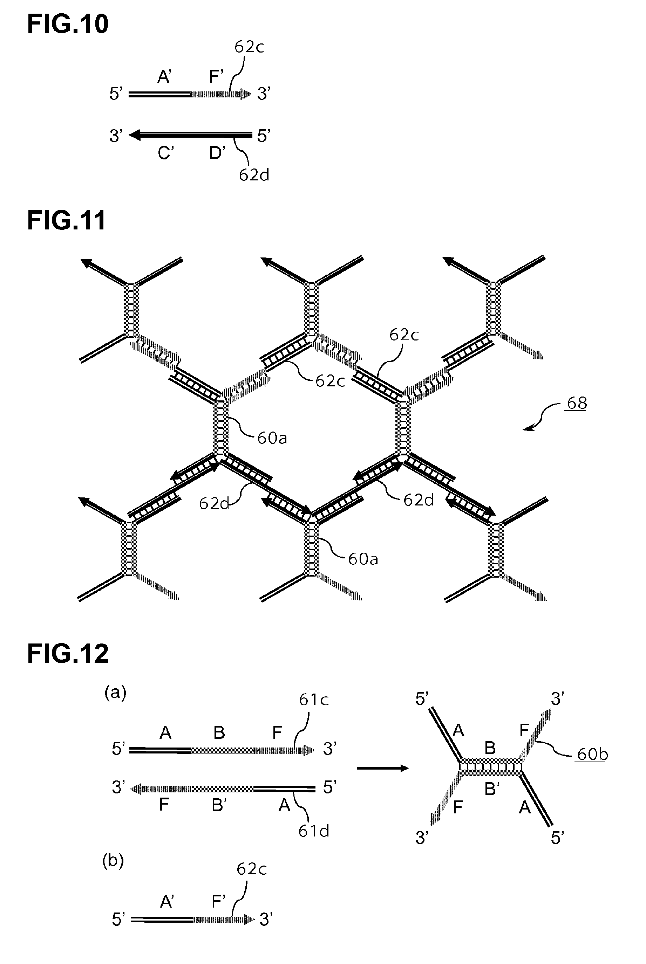

FIG. 10 is a schematic explanatory diagram illustrating another example (second example) of the pair of cross-linking probes (a first cross-linking probe 62c and a second cross-linking probe 62d) to be used together with the pair of dimer formation probes 61a, 61b illustrated in FIGS. 8.

As illustrated in FIG. 10, as another example of the pair of cross-linking probes, there is given a pair of cross-linking probes in which: the 5' side region of the first cross-linking probe 62c is complementary to the 5' side region (region A) of the first dimer formation probe 61a; the 3' side region of the first cross-linking probe 62c is complementary to the 3' side region (region F) of the second dimer formation probe 61b; the 5' side region of the second cross-linking probe 62d is complementary to the 5' side region (region D) of the second dimer formation probe 61b; and the 3' side region of the second cross-linking probe 62d is complementary to the 3' side region (region C) of the first dimer formation probe 61a. The dimer formation probes 61a, 61b illustrated in FIG. 8(a) and the cross-linking probes 62c, 62d illustrated in FIG. 10 can be allowed to hybridize with each other to form the signal probe polymer 68 (FIG. 11).

In the case of using a plurality of pairs of dimer formation probes, the same number of pairs of cross-linking probes are preferably used. Specifically, n (n represents an integer of 2 or more) pairs of dimer formation probes (i.e., 2n dimer formation probes) and n pairs of cross-linking probes (i.e., 2n cross-linking probes) are used, and there is suitably adopted such a configuration that: each of the 5' side regions of the dimer formation probes is complementary to any one of the 5' side regions of the cross-linking probes; each of the 5' side regions of the cross-linking probes is complementary to any one of the other 5' side regions of the dimer formation probes; each of the 3' side regions of the dimer formation probes is complementary to any one of the 3' side regions of the cross-linking probes; and each of the 3' side regions of the cross-linking probes is complementary to any one of the 3' side regions of the dimer formation probes.

FIGS. 14(c) and 14(d) are schematic explanatory diagrams illustrating an example of two pairs of cross-linking probes 72a to 72d to be used together with the two pairs of dimer formation probes 71a to 71d illustrated in FIGS. 14(a) and 14(b). FIG. 14(c) illustrates a first pair of cross-linking probes (the first cross-linking probe 72a and the second cross-linking probe 72b), and FIG. 14(d) illustrates a second pair of cross-linking probes (the first cross-linking probe 72c and the second cross-linking probe 72d).

As illustrated in FIGS. 14, two pairs of cross-linking probes (i.e., four kinds of cross-linking probes) are used as cross-linking probes to be used together with the two pairs of dimer formation probes 71a to 71d, and there is suitably adopted such a configuration that: each of the 5' side regions of the dimer formation probes is complementary to any one of the 5' side regions of the cross-linking probes; each of the 5' side regions of the cross-linking probes is complementary to any one of the 5' side regions of the dimer formation probes; each of the 3' side regions of the dimer formation probes is complementary to any one of the 3' side regions of the cross-linking probes; and each of the 3' side regions of the cross-linking probes is complementary to any one of the 3' side regions of the dimer formation probes.

Specifically, as illustrated in FIGS. 14, there are suitably used two pairs of cross-linking probes in which: the 5' side region of the first cross-linking probe 72a in the first pair is complementary to the 5' side region (region A) of the first dimer formation probe 71a in the first pair; the 5' side region of the second cross-linking probe 72b in the first pair is complementary to the 5' side region (region D) of the second dimer formation probe 71b in the first pair; the 5' side region of the first cross-linking probe 72c in the second pair is complementary to the 5' side region (region G) of the first dimer formation probe 71c in the second pair; the 5' side region of the second cross-linking probe 72d in the second pair is complementary to the 5' side region (region I) of the second dimer formation probe 71d in the second pair; the 3' side region of the first cross-linking probe 72a in the first pair is complementary to any one of the 3' side regions of the four kinds of dimer formation probes 71a to 71d (in FIGS. 14, there is illustrated a case of being complementary to the region H); the 3' side region of the second cross-linking probe 72b in the first pair is complementary to any one of the 3' side regions of the four kinds of dimer formation probes 71a to 71d, except for the one selected for the first cross-linking probe 72a in the first pair (in FIGS. 14, there is illustrated a case of being complementary to the region J); the 3' side region of the first cross-linking probe 72c in the second pair is complementary to any one of the 3' side regions of the four kinds of dimer formation probes 71a to 71d, except for the ones selected for the first and second cross-linking probes 72a, 72b in the first pair (in FIGS. 14, there is illustrated a case of being complementary to the region C); and the 3' side region of the second cross-linking probe 72d in the second pair is complementary to one of the 3' side regions of the four kinds of dimer formation probes 71a to 71d, except for the ones selected for the first and second cross-linking probes 72a, 72b in the first pair and the first cross-linking probe 72c in the second pair (in FIGS. 14, there is illustrated a case of being complementary to the region F). The dimer formation probes 71a to 71d and cross-linking probes 72a to 72d illustrated in FIGS. 14 can be allowed to hybridize with each other to form the signal probe polymer 68.

It should be noted that in FIGS. 14, there is illustrated a case where: the 3' side region of the first cross-linking probe 72a in the first pair is complementary to the 3' side region of the first dimer formation probe 71c in the second pair; the 3' side region of the second cross-linking probe 72b in the first pair is complementary to the 3' side region of the second dimer formation probe 71b in the second pair; the 3' side region of the first cross-linking probe 72c in the second pair is complementary to the 3' side region of the first dimer formation probe 71a in the first pair; and the 3' side region of the second cross-linking probe 72d in the second pair is complementary to the 3' side region of the second dimer formation probe 71b in the first pair, but no limitation is imposed on a combination of the 3' side regions of the dimer formation probes to be complementary to each of the 3' side regions of the cross-linking probes.

The length of each complementary region of the dimer formation probes and the cross-linking probes is at least 5 bases, preferably at least 8 bases, more preferably from 10 bases to 100 bases, still more preferably from 12 to 30 bases in terms of the number of bases. In addition, the lengths of the complementary regions in the respective probes are desirably the same. It should be noted that in the signal amplification probes according to the third exemplary embodiment, any one of the 3' side regions (e.g., regions C, F, H, J) includes a poly(T) sequence, and a region complementary thereto (e.g., regions C', F', H', J') includes a poly(A) sequence.

In the present invention, non-complementary base sequences may be any base sequences that do not hybridize with each other. The non-complementary base sequences also encompass base sequences that are identical to each other.
FIGS. 12 are schematic explanatory diagrams illustrating a third example of a pair of dimer formation probes and one kind of pair of cross-linking probes to be used in the present invention. In FIG. 12(a), reference symbol 60b denotes a dimer probe, and there is illustrated an example of dimer formation using two kinds of dimer formation probes 61c, 61d in which the 3' side regions and the 5' side regions each have identical base sequences. That is, there is adopted such a configuration that in the dimer probe 60a of FIGS. 8, the region A and the region D have identical base sequences, and the region C and the region F have identical base sequences. With this configuration, as illustrated in FIG. 12(b), the pair of cross-linking probes to be used together with the dimer probe 60b are identical to each other, and one kind of cross-linking probe 62c is used. The dimer formation probes 61c, 61d and cross-linking probe 62c illustrated in FIGS. 12 can be allowed to hybridize with each other to form the signal probe polymer 68 (FIG. 13).

As a signal amplification probe according to a fourth exemplary embodiment to be used in the present invention, there is given one kind of nucleic acid probe capable of self-assembling. The nucleic acid probe is a nucleic acid probe that includes three or more nucleic acid regions, and there is adopted such a configuration that each of the nucleic acid regions in the nucleic acid probe includes a first region and a second region complementary to the first region so that the regions are adjacent to each other. With this configuration, one kind of nucleic acid probe alone can form a polymer of the nucleic acid probe. For example, a nucleic acid probe disclosed in Patent Document 7 is used. In the description of the present application, the nucleic acid region in the nucleic acid probe means a region including the first region and the second region. In addition, each of the first region and second region in each nucleic acid region is referred to as complementary region.

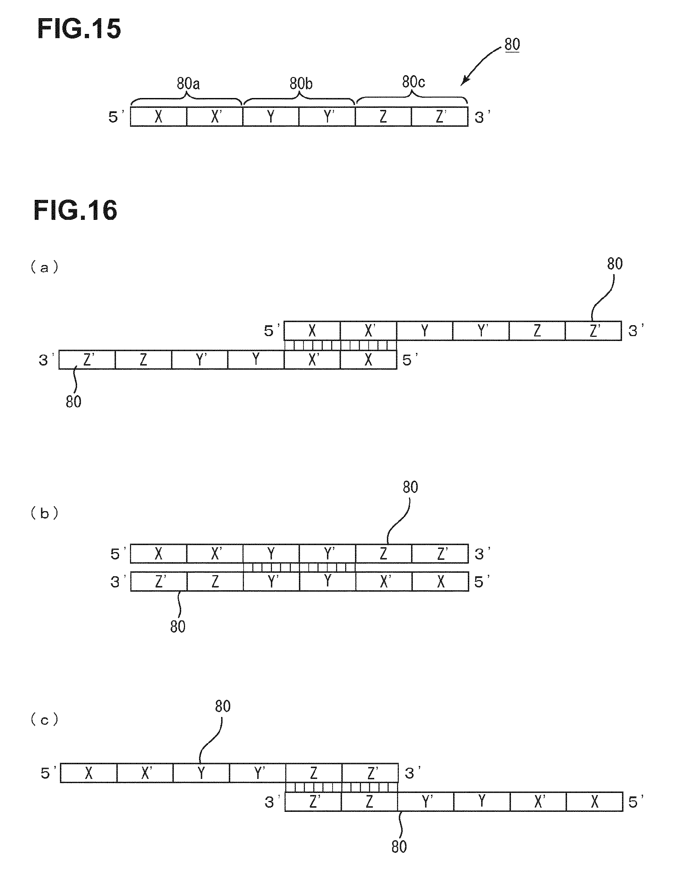

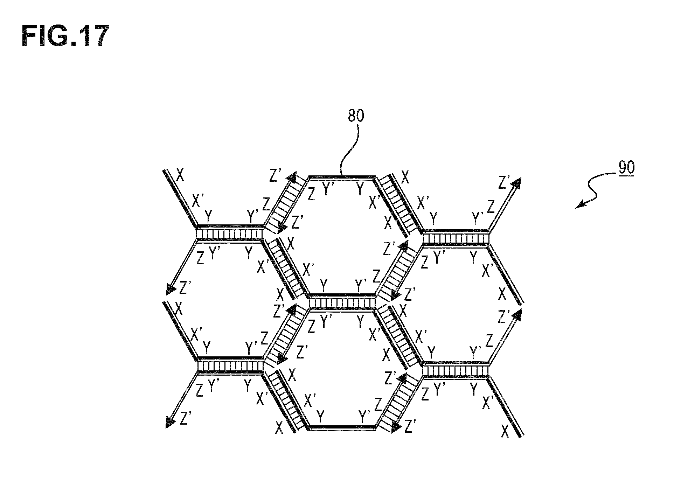

FIG. 15 is a schematic explanatory diagram illustrating an example of one kind of nucleic acid probe capable of self-assembling to be used in the present invention. FIGS. 16 are schematic explanatory diagrams illustrating the binding manner of the nucleic acid probes of FIG. 15. FIG. 17 is a schematic explanatory diagram illustrating a self-assembly (probe polymer) formed from the nucleic acid probes of FIG. 15.

FIG. 15 is a schematic explanatory diagram illustrating an example of the signal amplification probe according to the fourth exemplary embodiment to be used in the present invention, and there is illustrated an example including three nucleic acid regions. As illustrated in FIG. 15, one kind of nucleic acid probe 80 capable of self-assembling has a nucleic acid region 80a, a nucleic acid region 80b, and a nucleic acid region 80c in the stated order from the 5' end portion, and each of the nucleic acid regions 80a, 80b and 80c includes two regions complementary to each other so that the regions are adjacent to each other. That is, the nucleic acid region 80a includes a complementary region X and a complementary region X' having a base sequence complementary to the complementary region X so that the regions are adjacent to each other, the nucleic acid region 80b includes a complementary region Y and a complementary region Y' having a base sequence complementary to the complementary region Y so that the regions are adjacent to each other, and the nucleic acid region 80c includes a complementary region Z and a complementary region Z' having a base sequence complementary to the complementary region Z so that the regions are adjacent to each other.

In the nucleic acid probe 80, the complementary region X and the complementary region X', the complementary region Y and the complementary region Y', and the complementary region Z and the complementary region Z' are complementary nucleic acid regions capable of hybridizing with each other, respectively. As illustrated in FIGS. 16, the complementary region X and the complementary region X' bind to each other [FIG. 16(a)], the complementary region Y and the complementary region Y' bind to each other [FIG. 16(b)], and the complementary region Z and the complementary region Z' bind to each other [FIG. 16(c)]. It should be noted that in FIGS. 16, as a suitable example, there is illustrated a nucleic acid probe having three nucleic acid regions (80a, 80b, and 80c). However, the number of nucleic acid regions is not particularly limited as long as the number is 3 or more.

When the nucleic acid probes 80 are subjected to a hybridization reaction, as illustrated in FIG. 17, the nucleic acid probes 80 can self-assemble to form a probe polymer 90 as a self-assembly of the nucleic acid probes 80. It should be noted that the 3' region (e.g., region Z') of the signal amplification probe according to the fourth exemplary embodiment includes a poly(T) sequence, and a region complementary thereto (e.g., region Z) includes a poly(A) sequence.

In the nucleic acid probes 80, the length of each complementary region is preferably 2 bases or more, more preferably from 3 bases to 50 bases, still more preferably from 4 to 15 bases in terms of the number of bases. In addition, the lengths of the respective complementary regions in the nucleic acid probes are desirably the same.

In the present invention, it is suitable that in the respective signal amplification probes, the poly(T) sequence have a length of from 11 to 26 bases. It should be noted that in FIGS. 1, there is illustrated an example in which the entirety of the nucleic acid region Z is a poly(T) sequence. However, the first signal amplification probe 18a having the nucleic acid region Z including a poly(T) sequence and any other sequence may also be used.

The base sequence of each nucleic acid region of each signal amplification probe, except for the nucleic acid region including a poly(T) sequence, is not particularly limited as long as predetermined regions are configured to have complementary base sequences so as to form a probe polymer, but bases at both ends of each region are each preferably guanine or cytosine. When the bases at both ends of each region are each guanine or cytosine, a reaction time can be shortened, a stable probe polymer can be formed at additionally low reaction temperature, and workability and detection sensitivity can be improved.

The signal amplification probes are generally constructed of DNA or RNA, but may be constructed of a nucleic acid analog. Examples of the nucleic acid analog include a peptide nucleic acid (PNA, see, for example, WO 92/20702 A1) and a locked nucleic acid (LNA, see, for example, Koshkin AA et al. Tetrahedron 1998. 54, 3607-3630., Koshkin AA et al. J. Am. Chem. Soc. 1998. 120, 13252-13253., Wahlestedt C et al. PNAS. 2000. 97, 5633-5638.). In addition, the signal amplification probes of a pair are generally constructed of nucleic acids of the same kind, but for example, a DNA probe and an RNA probe may form a pair. That is, the kind of a nucleic acid of a probe may be selected from DNA, RNA and a nucleic acid analog (e.g., PNA or LNA). In addition, for nucleic acid composition in one probe, the probe does not need to be constructed of one kind of nucleic acid, for example, DNA alone, and for example, an oligonucleotide probe (chimeric probe) constructed of DNA and RNA may also be used as necessary, which is also included in the present invention.

Such probe may be synthesized by a known method. For example, the DNA probe may be synthesized by a phosphoramidite method using a DNA synthesizer Model 394 manufactured by Applied Biosystem Inc. In addition, a phosphoric acid triester method, an H-phosphonate method, a thiophosphonate method, or the like is available as an alternative method, and the probe may be synthesized by any method.

In the present invention, one or both of the respective signal amplification probes to be used are preferably labeled with a labeling substance 20. Suitable examples of the labeling substance 20 include a radioisotope, biotin, digoxigenin, a fluorophore, a luminophore and a pigment.

Next, as a method of detecting RNA according to a second aspect of the present invention, a case where the target RNA is RNA having no poly(A) tail, for example, microRNA is described below.

The method of detecting RNA according to the second aspect of the present invention, in which the target RNA is microRNA, is a method of detecting microRNA, the method including: (D) a poly(A) adding step of adding poly(A) to the 3' end of microRNA; (A) a capturing step of subjecting the microRNA and a capture substance for capturing the microRNA to a reaction to capture the microRNA; (B) a complex-for-detection forming step of subjecting the captured microRNA and one kind or a plurality of kinds of signal amplification probes to a reaction to form a complex for detection including the microRNA, the capture substance, and a probe polymer formed from the signal amplification probes; and (C) a detecting step of detecting the probe polymer bound in the complex for detection to detect the microRNA, the plurality of kinds of signal amplification probes being a plurality of kinds of signal amplification probes that have complementary base sequence regions capable of hybridizing with each other and can form a probe polymer through a self-assembly reaction, one of the plurality of kinds of signal amplification probes having a poly(T) sequence, the one kind of signal amplification probe being one kind of signal amplification probe having a poly(T) sequence and including three or more nucleic acid regions, each of the nucleic acid regions in the one kind of signal amplification probe including a first region and a second region complementary to the first region so that the regions are adjacent to each other.

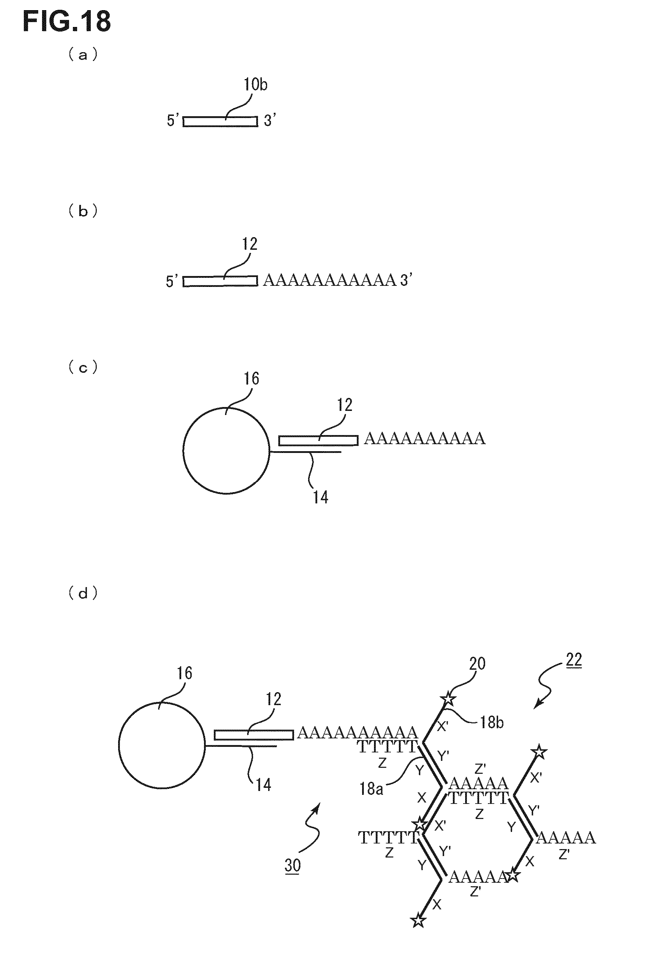

FIGS. 18 are schematic explanatory diagrams illustrating an example of the method of detecting RNA according to the second aspect of the present invention, FIG. 18(a) illustrates microRNA, FIG. 18(b) illustrates poly(A)-added microRNA after a poly(A) adding step, FIG. 18(c) illustrates microRNA captured by a capture substance after a capturing step, and FIG. 18(d) illustrates a complex for detection after a complex-for-detection forming step.

In FIGS. 18, reference symbol 10b denotes microRNA. The present invention is suitably used for the detection of a short target nucleic acid, is suitably used for the detection of microRNA having from 17 to 63 bases, and is particularly suitably used for the detection of microRNA having from about 17 to 24 bases. In addition, many kinds of nucleic acid molecules can be simultaneously detected.

As illustrated in FIG. 18(b), a poly(A) adding step of adding poly(A) to the 3' end of microRNA 10b is performed to form poly(A)-added microRNA 12 [step (D)]. Next, as illustrated in FIG. 18(c), a capturing step of subjecting the poly(A)-added microRNA 12 and the capture substance 14 for capturing the target microRNA 10b to a reaction to capture the poly(A)-added microRNA 12 is performed [step (A)]. It should be noted that in FIGS. 18, there is illustrated a case where the step (A) is performed after the step (D), but it may be possible to perform the step (D) after the step (A), that is, to perform a capturing step of subjecting the microRNA 10b and the capture substance 14 to a reaction to capture the microRNA 10b [step (A)], and then perform a poly(A) adding step of adding poly(A) to the 3' end of the microRNA 10b [step (D)] to capture the poly(A)-added microRNA 12 by the capture substance 14.

In the method of detecting RNA according to the second aspect of the present invention, the steps (A) to (C) may be performed in the same manner as in the above-mentioned first aspect.

In the present invention, a plurality of kinds of target nucleic acids can be simultaneously detected by using a plurality of the capture substances 14 in combination. In the simultaneous detection of the plurality of kinds of target nucleic acids, the respective signal amplification probes to be used may be the same, and the support 16 and the capture substance 14 only need to be prepared in a plurality of kinds, leading to excellent workability and low cost.

### Examples

The present invention is more specifically described below by way of Examples. However, it should be appreciated that Examples shown below are given for illustrative purposes and should not be interpreted as limiting the present invention.

### (Example 1) Detection of 12 kinds of microRNAs using synthetic RNA

Six kinds of synthetic oligoRNAs (hsa-miR-100, 155, 15b, 16, 21 and 23a/b) were used as target microRNAs. The six kinds of synthetic RNAs were mixed in equal amounts and used for an experiment with the amounts adjusted to 0, 0.316 amol, 1 amol, 3.16 amol, 10 amol or 31.6 amol.
Base sequence of hsa-miR-100
5'-AACCCGUAGAUCCGAACUUGUG-3' (SEQ ID NO: 1)
Base sequence of hsa-miR-155
5'-UUAAUGCUAAUCGUGAUAGGGG-3' (SEQ ID NO: 2)
Base sequence of hsa-miR-15b
5'-UAGCAGCACAUCAUGGUUUACA-3' (SEQ ID NO: 3)
Base sequence of hsa-miR-16
5'-UAGCAGCACGUAAAUAUUGGCG-3' (SEQ ID NO: 4)
Base sequence of hsa-miR-21
5'-UAGCUUAUCAGACUGAUGUUGA-3' (SEQ ID NO: 5)
Base sequence of hsa-miR-23a/b
5'-AUCACAUUGCCAGGGAUUUCC-3' (SEQ ID NO: 6)

12 kinds of spherical beads [fluorescent microparticles (Luminex bead, manufactured by Luminex Corporation.)] to which 12 kinds of capture probes (CP-hsa-let7d, CP-hsa-miR-100, 107, 155, 15b, 16, 181a, 181c, 21, 221, 23a/b and 92a) were bound, respectively, were used as capture substances.
Base sequence of CP-hsa-let7d
5'-AACTATGCAACCTACTACCTCT-3' (SEQ ID NO: 7)
Base sequence of CP-hsa-miR-100
5'-CACAAGTTCGGATCTACGGGTT-3' (SEQ ID NO: 8)
Base sequence of CP-hsa-miR-107
5'-TGATAGCCCTGTACAATGCTGCT-3' (SEQ ID NO: 9)
Base sequence of CP-hsa-miR-155
5'-CCCCTATCACGATTAGCATTAA-3' (SEQ ID NO: 10)
Base sequence of CP-hsa-miR-15b
5'-TGTAAACCATGATGTGCTGCTA-3' (SEQ ID NO: 11)
Base sequence of CP-hsa-miR-16
5'-CGCCAATATTTACGTGCTGCTA-3' (SEQ ID NO: 12)
Base sequence of CP-hsa-miR-181a
5'-ACTCACCGACAGCGTTGAATGTT-3' (SEQ ID NO: 13)
Base sequence of CP-hsa-miR-181c
5'-ACTCACCGACAGGTTGAATGTT-3' (SEQ ID NO: 14)
Base sequence of CP-hsa-miR-21
5'-TCAACATCAGTCTGATAAGCTA-3' (SEQ ID NO: 15)
Base sequence of CP-hsa-miR-221
5'-GAAACCCAGCAGACAATGTAGCT-3' (SEQ ID NO: 16)
Base sequence of CP-hsa-miR-23a/b
5'-GGAAATCCCTGGCAATGTGAT-3' (SEQ ID NO: 17)
Base sequence of CP-hsa-miR-92a
5'-ACAGGCCGGGACAAGTGCAATA-3' (SEQ ID NO: 18)

Two kinds of nucleic acid probes (HCP-1 and HCP-2) labeled with biotin at the 5' end were used as signal amplification probes.
Base sequence of HCP-1 (5'-X-Y-Z-3')
5'-CAACAATCAGGAC GATACCGATGAAG TTTTTTTTTTTTTTTTTTTT-3' (SEQ ID NO: 19)
Base sequence of HCP-2 (5'-X'-Y'-Z'-3')

5'-GTCCTGATTGTTG CTTCATCGGTATC
AAAAAAAAAAAAAAAAAAA-3' (SEQ ID NO: 20)

5 µL of the target microRNA, 1 µL of 10×PAP Buffer (manufactured by NEB), 1 µL of 10 mM ATP (manufactured by NEB), 0.2 µL of PolyA polymerase (manufactured by NEB), and 2.8 µL of D.W. were added so that the total volume was 10 µL, and the mixture was subjected to a reaction at 37°C for 15 minutes (poly(A) adding step).

To 10 µL of the reaction liquid after the poly(A) adding step were added 15 µL of a first hybridization reaction liquid [0.1 µL each of the 12 kinds of spherical beads (1,000 beads for each) (1.2 µL in total), 7.5 µL of 5 M tetra-methyl ammonium chloride (TMAC), 3.75 µL of 10×Supplement [500 mM Tris-HCl (pH 8.0), 40 mM EDTA (pH 8.0), 1% sarkosyl], and 2.55 µL of D.W.] so that the total volume was 25 µL, and the mixture was subjected to a reaction at 50°C for 60 minutes (capturing step).

To 25 µL of the reaction liquid after the capturing step were added 25 µL of a PALSAR reaction liquid [7.5 µL of 5 M TMAC, 2.5 µL of 10×Supplement, 2.5 µL of 20 pmol/µL HCP-1, 2.0 µL of 20 pmol/µL HCP-2 and 10.5 µL of D.W.] so that the total volume was 50 µL, and the mixture was subjected to a reaction at 44°C for 60 minutes (complex-for-detection forming step).

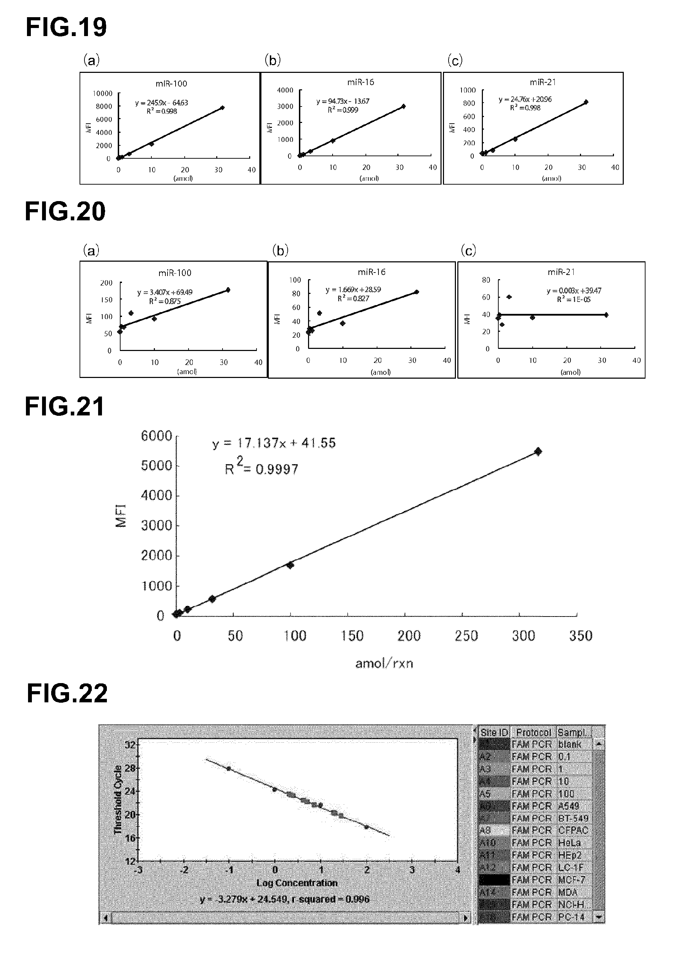

The reaction liquid after the complex-for-detection forming step was washed once with Wash Buffer [1×PBS with 0.02% tween 20 and 1.5 ppm Proclin 300]. After that, 50 µL of a detection reagent [SA-PE (manufactured by ProZyme, Inc.): 5 ng/µL] were added thereto, and the mixture was left to stand at room temperature for 10 minutes and then washed once with Wash Buffer. After that, 100 µL of Wash Buffer were added thereto, and fluorescence from the biotin and the fluorescent microparticles was measured by flow cytometry (Luminex System manufactured by Luminex Corporation) to detect signals from the 12 kinds of microRNAs (detecting step). Table 1 and FIGS. 19 show the results.

**[Table 1]**

| Target miRNA concentration (amol) | Detection signal from target miRNA (MFI) | | | | | | | | | | | |
|---|---|---|---|---|---|---|---|---|---|---|---|---|
| | let-7d | miR-100 | miR-107 | miR-155 | miR-15b | miR-16 | miR-181a | miR-181c | miR-21 | miR-221 | miR-23a/b | miR-92a |
| 0 | 15.7 | 12.2 | 25.7 | 28.7 | 20.8 | 12.0 | 27.0 | 39.0 | 33.3 | 22.2 | 14.2 | 19.5 |
| 0.316 | 16.2 | 68.5 | 20.8 | 63.8 | 65.2 | 33.0 | 27.0 | 42.3 | 40.7 | 24.0 | 40.7 | 19.7 |
| 1 | 18.3 | 220.8 | 25.7 | 140.2 | 144.5 | 77.5 | 30.2 | 42.7 | 46.3 | 22.7 | 112.3 | 21.8 |
| 3.16 | 16.7 | 706.3 | 26.0 | 389.8 | 380.8 | 282.3 | 28.7 | 40.3 | 84.2 | 19.3 | 366.7 | 17.7 |
| 10 | 16.3 | 2154.8 | 25.3 | 1274.3 | 1292.3 | 881.3 | 26.2 | 43.8 | 252.3 | 18.7 | 1252.2 | 22.2 |
| 31.6 | 16.0 | 7783.0 | 23.8 | 4353.7 | 4542.0 | 2996.8 | 25.0 | 39.7 | 810.2 | 21.8 | 3842.3 | 20.0 |

### (Comparative Example 1)

MicroRNA detection was performed using a commercially available miRNA detection kit and the same target microRNAs as in Example 1.

The microRNA detection was performed using Vantage microRNA Labeling Kit (manufactured by Marligen) as a labeling reagent, Vantage miRNA Multiplex Detection Kit Pancreatic Cancer Panel 1 (manufactured by Marligen) as a detection reagent, and the same six kinds of target microRNAs as in Example 1. The composition of the reagents and a reaction method were based on the protocols included with the kits. Table 2 and FIGS. 20 show the results.

**[Table 2]**

| Target miRNA concentration (amol) | Detection signal from target miRNA (MFI) | | | | | | | | | | | | |
|---|---|---|---|---|---|---|---|---|---|---|---|---|---|
| | let-7d | miR-100 | miR-107 | miR-155 | miR-15b | miR-16 | miR-181a | miR-181c | miR-21 | miR-221 | miR-23a/b | miR-92a | 5.8S control |
| 0 | 34 | 55 | 43 | 67 | 33 | 23 | 95 | 70 | 35 | 37 | 46 | 61 | 52 |
| 0.316 | 39 | 70 | 56 | 76 | 45 | 29 | 111 | 85 | 39 | 39 | 62 | 56 | 55 |
| 1 | 31 | 69 | 51 | 69 | 40 | 26 | 95 | 73 | 28 | 32 | 54 | 62 | 56 |
| 3.16 | 59 | 110 | 97 | 97 | 86 | 52 | 129 | 101 | 60 | 66 | 110 | 88 | 118 |
| 10 | 36 | 92 | 57 | 78 | 57 | 37 | 104 | 73 | 36 | 37 | 59 | 66 | 53 |
| 31.6 | 40 | 178 | 63 | 104 | 66 | 83 | 113 | 85 | 39 | 47 | 79 | 69 | 74 |

### (Example 2) Detection of miRNA using synthetic RNA and cell-derived total RNA

The synthetic oligoRNA (hsa-miR-21) used in Example 1 was used as target microRNA. The synthetic oligoRNA was used for an experiment with the amount adjusted to 0, 0.1 amol, 0.316 amol, 1 amol, 3.16 amol, 10 amol, 31.6 amol, 100 amol, or 316 amol (Example 2-1).

In addition, 10 ng each of 10 kinds of cell-derived total RNAs (NCI-H650, MCF-7, CFPAC-1, LC-1F, A549, Hep-2, PC-14, HeLa, MDA-MB-435s and BT549) were used as target microRNAs (Examples 2-2 to 2-11).

Spherical beads to which the capture probe (CP-hsa-miR-21) used in Example 1 was bound were used as a capture substance.

The poly(A) adding step was performed by the same method as in Example 1 using the above-mentioned target microRNA.

To 10 µL of the reaction liquid after the poly(A) adding step were added 15 µL of a first hybridization reaction liquid [0.4 µL of spherical beads (1,000 beads), 7.5 µL of 5 M TMAC, 3.75 µL of 10×Supplement [500 mM Tris-HCl (pH 8.0), 40 mM EDTA (pH 8.0), 1% sarkosyl] and 3.35 µL of D.W.] so that the total volume was 25 µL, and the mixture was subjected to a reaction at 50°C for 60 minutes (capturing step).

The complex-for-detection forming step was performed by the same method as in Example 1 using 25 µL of the reaction liquid after the capturing step.

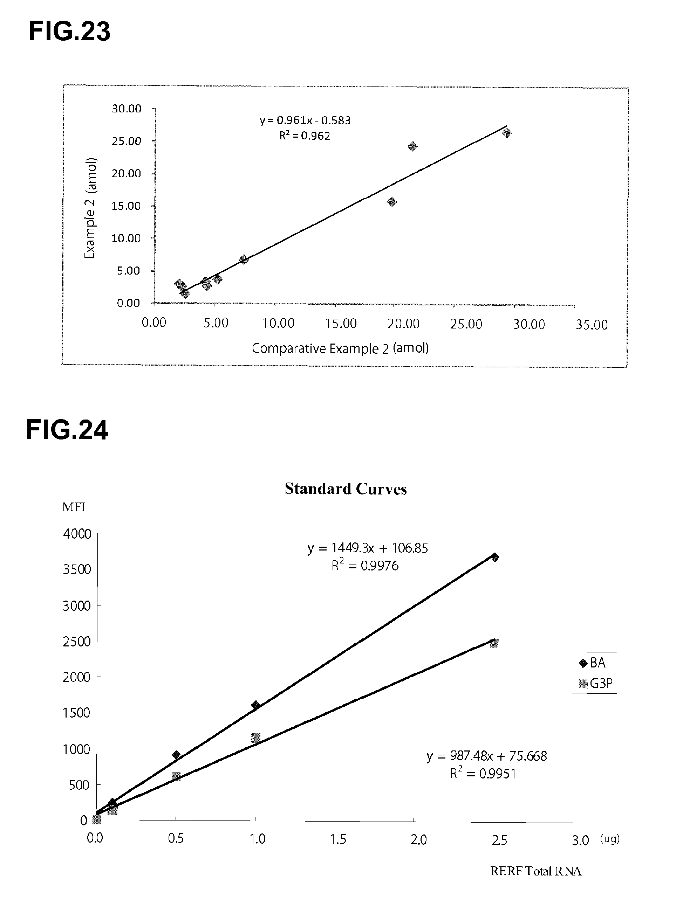

The reaction liquid after the complex-for-detection forming step was washed once with Wash Buffer [1×PBS with 0.02% tween 20, 0.1% BSA and 0.05% NaN₃]. After that, 50 µL of a detection reagent [SA-PE (manufactured by Invitrogen): 10 ng/µL] were added thereto, and the mixture was left to stand at room temperature for 10 minutes and then washed once with Wash Buffer. After that, 100 µL of Wash Buffer were added thereto, and fluorescence from the biotin and the fluorescent microparticles was measured by flow cytometry (Luminex System manufactured by Luminex Corporation) to detect signals from the microRNA (detecting step). FIG. 21 shows the results of Example 2-1. In addition, Table 3 shows the results of quantitative determination values of Example 2-2 to Example 2-11 calculated from the graph of FIG. 21. Further, FIG. 23 is a graph showing a correlation between the results of Example 2 and Comparative Example 2.

**[Table 3]**

| | Comparative Example 2 | Example 2 |
|---|---|---|
| A549 | 5.27 | 3.59 |
| BT-549 | 2.57 | 1.34 |
| CFPAC | 19.75 | 15.69 |
| HeLa | 2.09 | 2.86 |
| HEp2 | 4.25 | 3.18 |
| LC-1F | 7.44 | 6.62 |
| MCF-7 | 29.26 | 26.52 |
| MDA | 2.27 | 2.45 |
| NCI-H650 | 21.44 | 24.27 |
| PC-14 | 4.38 | 2.59 |
| (amol) | | |

### (Comparative Example 2)

MicroRNA (hsa-miR-21 and hsa-miR-16) detection was performed by a real-time PCR method using TaqManProbe and the same target microRNA as in Example 2.

A reverse transcription reaction was performed using TaqMan MicroRNA Primer (manufactured by ABI, for miR-21 and 16) and TaqMan MicroRNA Reverse Transcription Kit (manufactured by ABI) in accordance with the accompanying protocols. After that, a real-time PCR reaction was performed using TaqMan MicroRNA Assays (manufactured by ABI, for miR-21 and 16) and Universal PCR Master Mix, No AmpErase UNG (manufactured by ABI) and a reverse transcription reaction liquid in accordance with the accompanying protocols. FIG. 22 and Table 3 show the results. In addition, FIG. 23 is a graph showing a correlation between the results of Example 2 and Comparative Example 2.

As shown in the results of Examples 1 and 2, the invention of the present application allowed the microRNAs to be detected at a level of up to several amol, and allowed the microRNAs to be detected with high sensitivity and simply. Further, as shown in the results of Example 2 and Comparative Example 2, it was demonstrated that the invention of the present application exhibited a high correlation with the real-time PCR method.

### (Example 3) Detection of two kinds of messenger RNAs using culture cell-derived total RNA

Cell-derived total RNA (cell line RERF-LC-AI) including Beta-Actin (BA) and GAPDH (G3P) as target messenger RNAs was used. The total RNA was used for an experiment with the amount adjusted to 0, 0.1 µg, 0.5 µg, 1 µg or 2.5 µg.

Two kinds of spherical beads [fluorescent microparticles (Luminex bead, manufactured by Luminex Corporation)] to which two kinds of capture probes (CP-BA-1 and CP-G3P-1) were bound, respectively, were used as capture substances.
Base sequence of CP-BA-1 
5'-AAGGTGTGCACTTTATTCAACTGGTCTCAAGTCAGTGTACAGGTAA
GCCCTGGCTGCCTC-3' (SEQ ID NO: 21)
 Base sequence of CP-G3P-1

5'-GGTTGAGCACAGGGTACTTTATTGATGGTACATGACAAGGTGCGGCT
CCCTAGGCCCCTCC-3' (SEQ ID NO: 22)

The two kinds of nucleic acid probes (HCP-1 and HCP-2) labeled with biotin at the 5' end used in Example 1 were used as signal amplification probes.

To 10 µL of the total RNA were added 15 µL of a first hybridization reaction liquid [0.2 µL each of the two kinds of spherical beads (2,000 beads for each) (0.4 µL in total), 7.5 µL of 5 M tetra-methyl ammonium chloride (TMAC), 2.5 µL of 10×Supplement [500 mM Tris-HCl (pH 8.0), 40 mM EDTA (pH 8.0), 1% sarkosyl] and 4.6 µL of D.W.] so that the total volume was 25 µL, and the mixture was subjected to a reaction at 55°C for 60 minutes (capturing step).

To 25 µL of the reaction liquid after the capturing step were added 25 µL of a PALSAR reaction liquid [7.5 µL of 5 M TMAC, 2.5 µL of 10×Supplement, 2.5 µL of 20 pmol/µL HCP-1, 2.0 µL of 20 pmol/µL HCP-2 and 10.5 µL of D.W.] so that the total volume was 50 µL, and the mixture was subjected to a reaction at 44°C for 60 minutes (complex-for-detection forming step).

The reaction liquid after the complex-for-detection forming step was washed once with Wash Buffer [1×PBS with 0.02% tween 20 and 0.5% Sodium Azide]. After that, 50 µL of a detection reagent [SA-PE (manufactured by ProZyme, Inc.): 5 ng/µL] were added thereto, and the mixture was left to stand at room temperature for 10 minutes and then washed once with Wash Buffer. After that, 100 µL of Wash Buffer were added thereto, and fluorescence from the biotin and the fluorescent microparticles was measured by flow cytometry (Luminex System manufactured by Luminex Corporation) to detect signals from the two kinds of messenger RNAs (detecting step). FIG. 24 shows the results.

### (Example 4) Study on ratios of two kinds of messenger RNAs using five kinds of culture cell-derived total RNAs

Cell-derived total RNAs (cell lines RERF-LC-AI, A549, HeLa, PC-14 and Hep-2) including Beta-Actin and GAPDH as target messenger RNAs were used. The total RNAs were each used for an experiment with the amount adjusted to 1 µg or 5 µg.

Two kinds of spherical beads [fluorescent microparticles (Luminex bead, manufactured by Luminex Corporation)] to which the same two kinds of capture probes (CP-BA-1 and CP-G3P-1) as in Example 3 were bound, respectively, were used as capture substances.

The same two kinds of nucleic acid probes (HCP-1 and HCP-2) labeled with biotin at the 5' end as in Example 1 were used as signal amplification probes.

To 10 µL of the total RNA were added 15 µL of a first hybridization reaction liquid [0.2 µL each of the two kinds of spherical beads (2,000 beads for each) (0.4 µL in total), 7.5 µL of 5 M tetra-methyl ammonium chloride (TMAC), 2.5 µL of 10×Supplement [500 mM Tris-HCl (pH 8.0), 40 mM EDTA (pH 8.0), 1% sarkosyl] and 4.6 µL of D.W.] so that the total volume was 25 µL, and the mixture was subjected to a reaction at 55°C for 60 minutes (capturing step).

To 25 µL of the reaction liquid after the capturing step were added 25 µL of a PALSAR reaction liquid [7.5 µL of 5 M TMAC, 2.5 µL of 10×Supplement, 2.5 µL of 20 pmol/µL HCP-1, 2.0 µL of 20 pmol/µL HCP-2 and 10.5 µL of D.W.] so that the total volume was 50 µL, and the mixture was subjected to a reaction at 44°C for 60 minutes (complex-for-detection forming step).

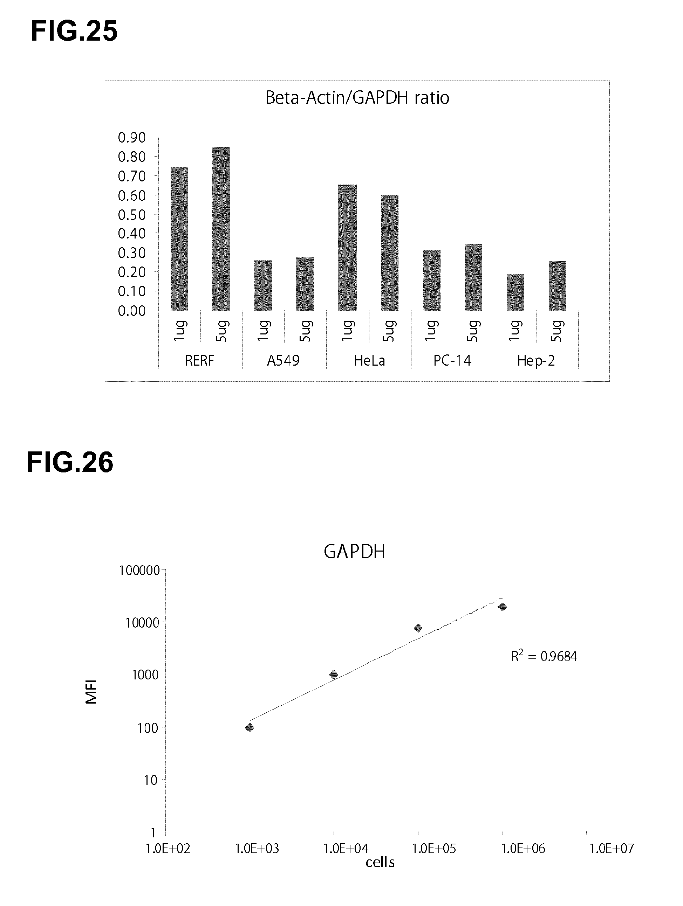

The reaction liquid after the complex-for-detection forming step was washed once with Wash Buffer [1×PBS with 0.1% BSA, 0.02% tween 20 and 0.5% Sodium Azide]. After that, 50 µL of a detection reagent [SA-PE (manufactured by Invitrogen): 10 ng/µL] were added thereto, and the mixture was left to stand at room temperature for 10 minutes and then washed once with Wash Buffer. After that, 100 µL of Wash Buffer were added thereto, and fluorescence from the biotin and the fluorescent microparticles was measured by flow cytometry (Luminex System manufactured by Luminex Corporation) to detect signals from the two kinds of messenger RNAs (detecting step). FIG. 25 shows the results.

### (Example 5) Detection sensitivity of messenger RNA using culture cell-derived total RNA

Cell-derived total RNA (cell line CCRF-CEM) including GAPDH as target messenger RNA was used. The total RNA extracted from 10⁷ cells was diluted and used for an experiment with the amount adjusted to one corresponding to 10³, 10⁴, 10⁵ or 10⁶ cells.

Spherical beads [fluorescent microparticles (Luminex bead, manufactured by Luminex Corporation)] to which a capture probe (CP-G3P-2) was bound were used as a capture substance.
Base sequence of CP-G3P-2

5'-TGGTGGTGCAGGAGGCATTGCTGATGATCTTGAGGCTGTTGTCATAC
TTCTCATGGTTCAC-3' (SEQ ID NO: 23)

In addition, the following two kinds of probes were used for the capture stabilization of RNA.
BL-1 
5'-ACCCATGACGAACATGGGGGCATCAGCAGAGGGGGCAGAGATGATG
ACCCTTTTGGCTCC-3' (SEQ ID NO: 24)
 BL-2

5'-TGAGTCCTTCCACGATACCAAAGTTGTCATGGATGACCTTGGCCAGG
GGTGCTAAGCAGT-3' (SEQ ID NO: 25)

The two kinds of nucleic acid probes (HCP-1 and HCP-2) labeled with biotin at the 5' end used in Example 1 were used as signal amplification probes.

To 10 µL of the total RNA were added 15 µL of a first hybridization reaction liquid [0.1 µL of the spherical beads (1,000 beads for each), 7.5 µL of 5 M tetra-methyl ammonium chloride (TMAC), 2.5 µL of 10×Supplement [500 mM Tris-HCl (pH 8.0), 40 mM EDTA (pH 8.0), 1% sarkosyl] and D.W. in such an amount that the total amount was adjusted to 15 µL] so that the total volume was 25 µL, and the mixture was subjected to a reaction at 55°C for 4 hours (capturing step).

To 25 µL of the reaction liquid after the capturing step were added 25 µL of a PALSAR reaction liquid [7.5 µL of 5 M TMAC, 2.5 µL of 10×Supplement, 2.5 µL of 20 pmol/µL HCP-1, 1.75 µL of 20 pmol/µL HCP-2 and 10.75 µL of D.W.] so that the total volume was 50 µL, and the mixture was subjected to a reaction at 44°C for 60 minutes (complex-for-detection forming step).

The reaction liquid after the complex-for-detection forming step was washed once with Wash Buffer [1×PBS with 0.02% tween 20 and 1.5 ppm ProClin 300]. After that, 50 µL of a detection reagent [SA-PE (manufactured by ProZyme, Inc.): 5 ng/µL] were added thereto, and the mixture was left to stand at room temperature for 10 minutes and then washed once with Wash Buffer. After that, 100 µL of Wash Buffer were added thereto, and fluorescence from the biotin and the fluorescent microparticles was measured by flow cytometry (Luminex System manufactured by Luminex Corporation) to detect signals from the messenger RNA (detecting step). FIG. 26 shows the results.

As shown in the results of Examples 3 and 4, in the invention of the present application, the messenger RNA was successfully detected in an intact and non-labeled state of the RNA without performing a reverse transcription reaction or a PCR. In addition, irrespective of the amounts of a target to be used, a housekeeping gene present at a certain ratio in each cell was able to be detected. Further, as shown in the results of Example 5, it was demonstrated that the messenger RNA of GAPDH was able to be detected with linearity and high sensitivity from 10³ cells.

### Reference Signs List

10a: messenger RNA, 10b: microRNA, 12: poly(A)-added microRNA, 14: capture substance, 16: support, 18a: first signal amplification probe , 18b: second signal amplification probe, 20: labeling substance, 22, 50, 68, 90: probe polymer, 30: complex for detection, 40a: first dimer probe, 40b, 40c, 40d: second dimer probe, 40e: third dimer probe, 41a to 41h, 41j, 41k: dimer formation probe, 60a, 60b: dimer probe, 61a to 61d: dimer formation probe, 62a to 62d: cross-linking probe, 70a, 70b: dimer probe, 71a to 71d: dimer formation probe, 72a to 72d: cross-linking probe, 80: nucleic acid probe, 80a, 80b, 80c: nucleic acid region.

## Claims

1. A method of detecting RNA, including detecting target RNA,
the method comprising:
(A) a capturing step of capturing RNA by a capture substance for capturing the RNA;
(B) a step of subjecting the captured RNA, a first signal amplification probe, and a second signal amplification probe to a reaction to form a complex for detection comprising the RNA, the capture substance, and a probe polymer formed from the first signal amplification probe and the second signal amplification probe; and
(C) a step of detecting the probe polymer bound in the complex for detection to detect the RNA,
the first signal amplification probe comprising a nucleic acid probe that comprises at least a nucleic acid region X, a nucleic acid region Y, and a nucleic acid region Z comprising a poly(T) sequence in the stated order from a 5' end side,
the second signal amplification probe comprising a nucleic acid probe that comprises at least a nucleic acid region X' complementary to the nucleic acid region X, a nucleic acid region Y' complementary to the nucleic acid region Y, and a nucleic acid region Z' complementary to the nucleic acid region Z in the stated order from a 5' end side.

2. The method of detecting RNA according to claim 1, wherein in the first signal amplification probe, the poly(T) sequence has a length of from 11 to 26 bases.

3. The method of detecting RNA according to claim 1 or 2, wherein in the first signal amplification probe, the nucleic acid region X has a length of from 11 to 20 bases, the nucleic acid region Y has a length of from 11 to 20 bases, and the nucleic acid region Z has a length of from 11 to 26 bases.

4. The method of detecting RNA according to any one of claims 1 to 3, wherein the first signal amplification probe and/or the second signal amplification probe is labeled with a labeling substance.

5. The method of detecting RNA according to any one of claims 1 to 4, wherein the capture substance comprises a base sequence complementary to the RNA, and is immobilized onto a support or has introduced therein a moiety capable of being immobilized onto the support.

6. The method of detecting RNA according to any one of claims 1 to 5, wherein the target RNA comprises messenger RNA.

7. The method of detecting RNA according to any one of claims 1 to 5, wherein the target RNA comprises microRNA, and the method further comprises (D) a poly(A) adding step of adding poly(A) to a 3' end of the microRNA.

8. A kit for detecting RNA,
the kit comprising:
a capture substance for capturing target RNA;
a first signal amplification probe; and
a second signal amplification probe,
wherein the first signal amplification probe comprises a nucleic acid probe that comprises at least a nucleic acid region X, a nucleic acid region Y, and a nucleic acid region Z comprising a poly(T) sequence in the stated order from a 5' end side, and
wherein the second signal amplification probe comprises a nucleic acid probe that comprises at least a nucleic acid region X' complementary to the nucleic acid region X, a nucleic acid region Y' complementary to the nucleic acid region Y, and a nucleic acid region Z' complementary to the nucleic acid region Z in the stated order from a 5' end side.

9. A pair of signal amplification probes, comprising:
a first signal amplification probe; and
a second signal amplification probe,
wherein the first signal amplification probe comprises a nucleic acid probe that comprises at least a nucleic acid region X, a nucleic acid region Y, and a nucleic acid region Z comprising a poly(T) sequence in the stated order from a 5' end side,
wherein the second signal amplification probe comprises a nucleic acid probe that comprises at least a nucleic acid region X' complementary to the nucleic acid region X, a nucleic acid region Y' complementary to the nucleic acid region Y, and a nucleic acid region Z' complementary to the nucleic acid region Z in the stated order from a 5' end side, and
wherein the pair of signal amplification probes is used in the method according to any one of claims 1 to 7.
